(19) Europäisches Patentamt
European Patent Office
Office européen des brevets

(11) EP 0 575 496 B1

(12) **FASCICULE DE BREVET EUROPEEN**

(45) Date de publication et mention de la délivrance du brevet: **27.08.1997 Bulletin 1997/35**

(21) Numéro de dépôt: **92908216.2**

(22) Date de dépôt: **13.03.1992**

(51) Int. Cl.$^6$: **A61K 7/06**, A61K 7/48, A61K 7/00

(86) Numéro de dépôt international: **PCT/FR92/00235**

(87) Numéro de publication internationale: **WO 92/16186 (01.10.1992 Gazette 1992/25)**

(54) **COMPOSITION COSMETIQUE OU PHARMACEUTIQUE, NOTAMMENT DERMATOLOGIQUE, CONTENANT DE L'OXYACANTHINE, EN PARTICULIER DESTINEE A STIMULER LA POUSSE DES CHEVEUX OU A RETARDER LEUR CHUTE**

OXYACANTHIN ENTHALTENDE KOSMETISCHE ODER PHARMAZEUTISCHE INSBESONDERE DERMATOLOGISCHE ZUSAMMENSETZUNG ZUR STIMULIERUNG DES HAARWACHSTUMS ODER VERZOEGERUNG DES HAARAUSFALLS

COSMETIC OR PHARMACEUTICAL, ESPECIALLY DERMATOLOGICAL, COMPOSITION,CONTAINING OXYACANTHINE, IN PARTICULAR FOR STIMULATING HAIR GROWTH OR RETARDING HAIR LOSS

(84) Etats contractants désignés:
**BE CH DE ES FR GB GR IT LI MC NL SE**

(30) Priorité: **14.03.1991 FR 9103120**

(43) Date de publication de la demande:
**29.12.1993 Bulletin 1993/52**

(73) Titulaire: **LVMH RECHERCHE**
**92752 Nanterre (FR)**

(72) Inventeurs:
- **BONTE, Frédéric**
  **F-92400 Courbevoie (FR)**
- **MEYBECK, Alain**
  **F-92400 Courbevoie (FR)**

(74) Mandataire: **Portal, Gérard**
**Cabinet Beau de Loménie**
**158, rue de l'Université**
**75340 Paris Cédex 07 (FR)**

(56) Documents cités:

| | |
|---|---|
| **EP-A- 0 087 993** | **EP-A- 0 107 559** |
| **FR-A- 2 510 402** | **FR-A- 2 609 390** |
| **GB-A- 2 050 166** | **GB-A- 2 136 812** |

- **PATENT ABSTRACTS OF JAPAN vol. 14, no. 211 (C-715)(4154) 2 Mai 1990**
- **PATENT ABSTRACTS OF JAPAN vol. 8, no. 199 (C-242)(1636) 12 Septembre 1984**
- **PATENT ABSTRACTS OF JAPAN vol. 10, no. 71 (C-334)(2128) 20 Mars 1986**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 575 496 B1

Printed by Rank Xerox (UK) Business Services
2.14.12/3.4

## Description

La présente invention concerne essentiellement une composition cosmétique ou pharmaceutique, notamment dermatologique, contenant de l'oxyacanthine, en particulier destinée à stimuler la pousse des cheveux ou à retarder leur chute. L'oxyacanthine peut être utilisée telle quelle ou sous forme de dérivés, en particulier un dérivé acylé, tel que le dérivé acétyle ou propionyle, ou un dérivé alkylé tel que méthyle, ou de ses sels, en particulier ses sels d'addition d'acide. En particulier, il s'agit des dérivés au niveau du groupe hydroxy phénolique de l'oxyacanthine, qui est généralement numéroté en position 12' du squelette oxyacanthane, selon la nomenclature utilisée dans Chemical Abstracts. L'oxyacanthine peut être utilisée seule ou en combinaison avec au moins une saponine. L'oxyacanthine peut être extraite d'une plante, notamment choisie parmi les espèces suivantes : Berberis, Mahonia, Laurelia, Cocculus et Xanthorhiza, ou peut se présenter sous forme d'extrait de cette plante.

En particulier, les plantes du genre Berberis sont bien connues notamment étant donné leur caractère commun et le fait qu'elles poussent sur la plus grande partie de l'Europe, notamment en Angleterre, en Afrique du Nord, et en Asie tempérée. C'est une plante qui est également utilisée dans les jardins à titre ornemental. Il est connu que les plantes du genre Berberis contiennent de nombreuses substances actives et principalement de la berbérine. Parmi les autres substances, on connait l'oxyacanthine.

L'oxyacanthine a déjà été décrite comme ayant un effet antitumoral dans le document Chem. Pharm. Bull., (1976), vol. 24, n° 10, pages 2413-20, et comme agent actif dans les maladies hépatobiliaires, voir le document RO-63427.

Le document Patent Abstracts of Japan, volume 14, n° 211 (C-715) (4154) de mai 1990 et JP-A-2148517 (SHISEIDO COMPANY LIMITED), 19 février 1990, décrit une lotion capillaire destinée à stimuler la pousse des cheveux ou à retarder leur chute contenant au moins un extrait de diverses plantes en combinaison avec un ou plusieurs agents tensioactifs comme ingrédients essentiels. Parmi les plantes, il est cité la plante Berberis Thunbergii. Ce document ne contient aucune mention relativement à l'oxyacanthine.

De même, le document FR-A-2 510 402 décrit des compositions capillaires pour traiter la calvitie et pour redonner au cuir chevelu sa vigueur naturelle, constituées par du vinaigre de cidre et d'une association de plantes sécifiques parmi lesquelles la plante Mahonia. Ce document ne contient également aucune indication concernant l'oxyacanthine.

Selon la présente invention, il a été découvert de manière surprenante que l'oxyacanthine, ou ses dérivés ou sels, présentait une bonne efficacité sur la prolongation de la phase de pousse active du poil (phase anagène), et, par conséquent, pouvait ralentir la chute des cheveux. Il a été découvert, en outre, que l'oxyacanthine, ou ses dérivés ou sels, possédait une activité sur le prurit, notamment le prurit du cuir chevelu qui accompagne souvent les affections du cheveu.

Ainsi, la présente invention a pour but de résoudre le nouveau problème technique consistant en la fourniture d'une nouvelle formulation d'une composition cosmétique ou pharmaceutique, notamment dermatologique, présentant une bonne efficacité sur la pousse des cheveux, le ralentissement de leur chute, ainsi que sur le prurit.

La présente invention a également pour but de résoudre ce nouveau problème technique en proposant une nouvelle formulation d'efficacité accrue, par la mise en oeuvre d'effets de combinaison de substances actives.

La présente invention permet de résoudre ces nouveaux problèmes techniques pour la première fois, de manière satisfaisante, fiable, peu coûteuse et donc utilisable à l'échelle industrielle.

Ainsi, selon un premier aspect, la présente invention fournit une composition cosmétique, en particulier destinée à stimuler la pousse des cheveux, à retarder leur chute, ou à combattre le prurit, notamment le prurit du cuir chevelu, caractérisée en ce qu'elle comprend, à titre d'ingrédient actif, une quantité cosmétiquement efficace d'oxyacanthine, de l'un de ses dérivés ou sels, en particulier les dérivés au niveau du groupe hydroxy phénolique de l'oxyacanthine, de préférence de formule (I) suivante :

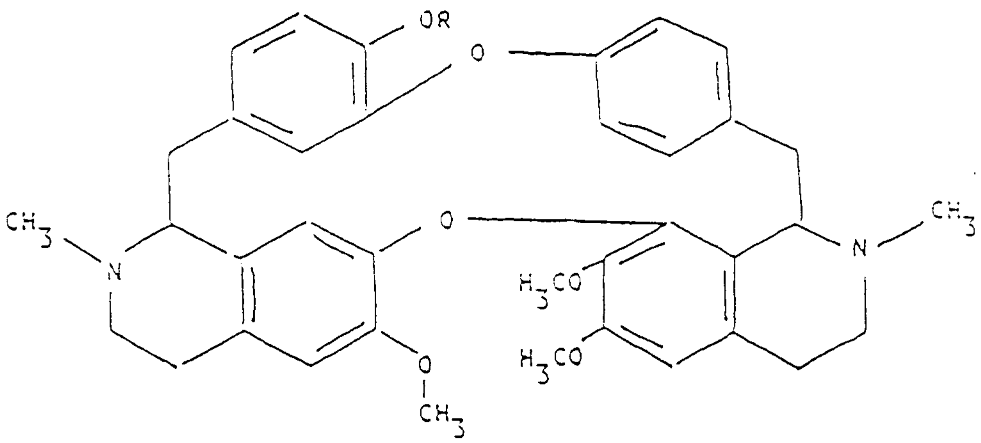

(I)

dans laquelle :

R représente un atome d'hydrogène (oxyacanthine) ou un radical acyle en $C_2$-$C_{12}$, ou une chaîne hydrocarbonée en $C_1$-$C_{12}$, ou un de ses sels d'addition d'acide, en particulier cosmétiquement acceptable, ou d'un extrait de plante contenant l'oxyacanthine ou l'un de ses dérivés tels que précédemment définis, à l'exclusion d'un extrait de la plante Berberis thunbergii, et de la plante Mahonia.

On notera que les dérivés d'oxyacanthine présentent généralement l'avantage d'être plus stables que l'oxyacanthine elle-même.

Selon une variante de réalisation particulière, la chaîne hydrocarbonée et le radical acyle peuvent être saturés ou non, linéaires, ramifiés ou en partie cycliques, notamment aromatiques.

Selon une variante de réalisation avantageuse, R représente en particulier un radical méthyle, hexyle, décyle, acétyle, propionyle, butanoyle, hexanoyle, octanoyle ; encore mieux méthyle, acétyle ou propionyle.

Une synthèse des dérivés alkylés a notamment été décrite dans le document JP-02-111 777 relatif à des agents anticancéreux.

Le dérivé méthylé (R=méthyle) peut être soit synthétisé suivant le procédé décrit plus loin, ou selon le procédé décrit par P. DUTE et al., dans Phytochemistry 1988, volume 27, n° 2, pages 655-657, soit encore extrait à partir de plante, notamment de Berberis cretica, Berberis pseudambalata, B. stolonifera, Berberis laurina, Laurelia sempervirens ou Mahonia repens.

Les dérivés acylés, tel qu'en particulier acétate et propanoate, peuvent être préparés selon un procédé classique d'acylation, tel que celui décrit par P. DUTE et al. dans Phytochemistry 1988, volume 27, n° 2, pages 655-657, ou dans le document JP-03-68 578.

En particulier, pour acétyler la fonction hydroxy phénolique de l'oxyacanthine, on dissout l'oxyacanthine dans du dichlorométhane, on ajoute 10 équivalents d'anhydride acétique, 1 équivalent de pyridine en présence également de p-diméthylaminopyridine comme catalyseur. Après dilution dans du dichlorométhane, on lave par de l'eau alcaline pour éliminer les pyridines, puis par de l'eau acide pour éliminer l'excès d'anhydride acétique. On concentre la phase organique, puis on prépare de manière classique le chlorhydrate de l'acétate d'oxyacanthine qui précipite.

Selon un mode particulier de réalisation, la composition telle que précédemment définie contient du sulfate d'oxyacanthine ou un mélange de sulfates de bases tertiaires incluant le sulfate d'oxyacanthine, ce mélange étant généralement préparé à partir d'un extrait de plante.

Suivant un autre mode de réalisation particulier de l'invention, l'extrait de plante précité est un extrait de plante choisie parmi les espèces suivantes : Berberis, en particulier :
B. amurensis Rupr., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib., B. Laurina, B. nummularia, B. oblonga, B. orthobotrys, B. paucidentata Rusby, B. polyantha Hemsl., B. poiretii Schneid., B. pseudambalata, B. sargentiana Schneid., B. silva-taroucana Schneid., B. soulicana Schneid., B. stolonifera, B. vernae Sehneid., B. vulgaris ou épine vinette, B. wilsonae Hemsl. ; Mahonia, en particulier M. repens, M. aquifolia ; Laurelia, en particulier L. sempervirens ; Cocculus, en particulier C. laurifolius; Xanthorhiza, en particulier X. simplicissima.

Les parties de plante utilisées pour la préparation dudit extrait sont les feuilles, les tiges, les racines, les baies, et de préférence l'écorce des tiges ou des racines.

Selon une variante préférée, l'extrait précité est un extrait de Berberis à l'exclusion de Berberis thunbergii, de préférence choisi parmi Berberis vulgaris, B. cretica, B. pseudambalata, B. stolonifera, B. laurina, en particulier un extrait d'écorce de racines de cette plante.

Selon un mode de réalisation avantageux, la concentration pondérale en oxyacanthine, en l'un de ses dérivés ou en l'un de ses sels d'addition d'acide ou d'extrait de plante en contenant précités, par rapport au poids total de la composition, est comprise entre 0,001 % et 5 %, de préférence entre 0,01 % et 2 %.

Selon un deuxième aspect, la présente invention fournit une composition cosmétique ou pharmaceutique, notamment dermatologique, en particulier destinée à stimuler la pousse des cheveux, à retarder leur chute, ou à combattre le prurit, notamment le prurit du cuir chevelu, caractérisée en ce qu'elle comprend, à titre d'ingrédient actif, une quantité cosmétiquement ou pharmaceutiquement efficace d'une association constituée par l'oxyacanthine, ses dérivés ou ses sels, de préférence de formule (I) précitée ou l'un de ses sels d'addition d'acide, en particulier cosmétiquement ou pharmaceutiquement acceptable, ou d'un extrait de plante en contenant, et au moins une saponine.

Selon un autre mode de réalisation, la présente invention fournit une composition cosmétique ou pharmaceutique, notamment dermatologique, en particulier destinée à stimuler la pousse des cheveux, à retarder leur chute ou à combattre le prurit, notamment le prurit du cuir chevelu, caractérisée en ce qu'elle comprend une quantité cosmétiquement ou pharmaceutiquement efficace d'une substance active constituée par l'oxyacanthine, ses dérivés ou ses sels, de préférence de formule (I) précitée ou l'un de ses sels d'addition d'acide, en particulier cosmétiquement ou pharmaceutiquement acceptable, ou un extrait de plante en contenant, en présence de phases lamellaires lipidiques hydratées ou de liposomes, contenant ou non ladite substance active.

Suivant un mode de réalisation particulier, le sel d'addition d'acide et l'extrait de plante précités sont tels que définis précédemment.

Suivant un mode de réalisation avantageux de l'invention, la saponine précitée est choisie parmi le groupe des saponines stéroïdiques, notamment les saponines à sapogénine du type "diosgénine" et celles à sapogénine du type "sarsapogénine", et des saponines triterpéniques, notamment les saponines à sapogénine de structure "oléanane", telles que l'acide oléanolique, l'acide médicagénique, le sapogénol ou l'hédéragénine, et celles à sapogénine de structure "ursane", telles que l'acide ursolique ou l'acide asiatique.

Selon une variante préférée, la saponine utilisée pour la mise en oeuvre de la présente invention est extraite de l'une des plantes suivantes : Tribulus terrestris, Dioscorea, Smilax excelsa, Paris polyphylla, Cornus florida, Yucca, Smilax aristolochiaefolia, Asparagus officinalis, Hedera helix, Medicago - en particulier Medicago sativa ou luzerne -, Centella asiatica, Trigonella fenugraecum.

Suivant une variante avantageuse, la composition précitée contient un mélange de saponines extrait de l'une des plantes précitées, en particulier de Medicago sativa.

Suivant un mode de réalisation avantageux, la concentration pondérale en oxyacanthine, ses dérivés ou en l'un de ses sels d'addition d'acide précités, par rapport au poids total de la composition, est comprise entre 0,001 % et 5 %, de préférence entre 0,01 % et 2 %.

Suivant un mode de réalisation avantageux de l'invention, les proportions relatives d'oxyacanthine ou de l'un de ses sels d'addition d'acide ou d'extrait de plante en contenant et d'une saponine ou d'un mélange de saponines précités peuvent varier dans de larges limites. Il est préféré des rapports relatifs de 10/1 à 1/10 en poids. De préférence, ce rapport est de 1/1 en poids.

Pour la mise en oeuvre de la présente invention, on peut utiliser de l'oxyacanthine disponible dans le commerce, en particulier sous forme de sel d'addition d'acide, tel que le sulfate disponible par exemple auprès de la Société EXTRASYNTHESE Genay-France.

Les dérivés d'oxyacanthine de formule (I) précitée peuvent être préparés de manière classique par des procédures de synthèse bien connues à l'homme de l'art dont les principales ont été précédemment énoncées. L'homme de l'art pourra également se reporter à l'exemple de synthèse donné plus loin.

On peut également obtenir l'oxyacanthine, ses dérivés, ou un sel d'addition à partir d'une plante, telle qu'une plante de l'une des espèces précitées, en particulier de l'espèce Berberis. Certains procédés d'obtention ont été décrits, par exemple par A. ORECHOW dans Archiv der Pharmazie (1933) 271, 323-27 ou par P. PETCU et T. GOINA dans Planta Medica (1970) 18 (4), 372-5.

En particulier, pour obtenir un mélange de bases tertiaires, incluant l'oxyacanthine, à partir d'une plante telle que précédemment définie, on peut opérer selon le procédé suivant. La matière végétale préalablement broyée est humectée par de l'ammoniaque à 25 %. Le tout est laissé en macération en présence d'acétate d'éthyle, à raison de 35 parties de solvant, pour une partie de matière végétale environ, pendant 24 h. On effectue ensuite une lixiviation avec l'acétate d'éthyle. On épuise ensuite la phase organique par une solution aqueuse diluée d'acide sulfurique à 2 %. La phase aqueuse obtenue est alors alcalinisée par l'ammoniaque en présence de chloroforme. On décante. La phase aqueuse est ensuite à nouveau extraite par du chloroforme. Les phases chloroformiques sont réunies, puis lavées à l'eau et séchées. Le chloroforme est ensuite évaporé et on obtient ainsi un mélange de bases tertiaires incluant l'oxyacanthine.

A partir de ce mélange, on peut préparer un sel d'addition d'acide de ces bases, notamment un sulfate, en opérant de la manière suivante.

On introduit le mélange de bases obtenu par le procédé précédemment décrit dans une quantité d'acétone suffisante pour le dissoudre. La solution est acidifiée à pH 3 par de l'acide sulfurique concentré, ce qui provoque la précipitation des bases sous forme de sulfates. Après filtration, les cristaux sont dissous dans du méthanol. La solution méthanolique est ensuite évaporée à sec. On obtient ainsi un mélange de sulfates de bases tertiaires, solubles dans l'eau, incluant le sulfate d'oxyacanthine.

Selon encore un mode de réalisation particulier de l'invention, l'extrait de plante précité contenant l'oxyacanthine est obtenu par le procédé d'extraction suivant. On extrait la matière sèche, constituée de préférence d'écorce de racine de la plante précédemment définie, au moyen d'un solvant choisi parmi le groupe constitué par : les alcools comportant de préférence de 1 à 4 atomes de carbone, les esters organiques comportant de préférence de 3 à 6 atomes de carbone ; ou d'un solvant mixte à base d'un mélange quelconque des solvants précités, ou d'un mélange hydro-alcoolique.

Avantageusement, le solvant d'extrait primaire est le méthanol, l'éthanol, l'acétate d'éthyle, un mélange méthanol-eau ou un mélange éthanol-eau.

Le rapport de la matière végétale à l'agent d'extraction est généralement compris entre 1:5 et 1:20 parties en poids, mais il est de préférence d'environ 1:10 parties en poids.

Par ailleurs, il peut être avantageux d'alcaliniser la matière sèche avant de procéder à l'extraction primaire, par exemple avec de l'ammoniaque.

L'extraction primaire s'effectue à des températures comprises entre la température ambiante et le point d'ébullition du solvant utilisé pour l'extraction.

De préférence, l'extraction primaire est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée et puis concentrée et/ou évaporée à sec sous pression réduite. On obtient ainsi un premier extrait selon l'invention contenant de l'oxyacanthine.

Dans le cas où le solvant primaire contient de l'eau, par exemple un mélange hydro-alcoolique à 30 % en poids d'alcool, on préfère éliminer le solvant, après filtration, par lyophilisation à -80°C.

Par ailleurs, lorsque selon la présente invention on associe l'oxyacanthine, ses dérivés ou l'un de ses sels d'addition d'acide ou un extrait de plante en contenant, à au moins une saponine, celle-ci peut également être obtenue par extraction à partir de plantes en particulier de celles citées plus haut. Des procédés d'extraction de saponines sont connus, en particulier par les documents suivants : US-3 351 582, US-3 449 760, US-3 620 919, DE-2 118 916, SU-403 409, GB-1 378 278, US-3 901 875, ou encore par la publication de G. Massiot et al. dans J. Agric. Food Chem. (1988) 36 (5) 902-909.

En ce qui concerne plus particulièrement l'extraction des saponines de Medicago, en particulier de Medicago sativa, on utilisera de préférence le procédé décrit par G. Massiot et al. dans le document précité, ou celui décrit dans la demande de brevet français FR 90-14542.

Selon une variante de réalisation, la composition cosmétique ou pharmaceutique, notamment dermatologique selon l'invention, comprend en outre au moins une autre substance active, à une concentration efficace, choisie parmi la quinine ou ses dérivés, les rubéfiants tels que le nicotinate de méthyle, un surnageant de culture de fibroblastes de papilles, tel que défini dans le document EP-A-272 920, des hydrolysats de kératine, des oligo-éléments tels que zinc, sélénium, cuivre, des inhibiteurs de 5-$\alpha$-réductase tels que : progestérone, cyprotérone acétate, Minoxidil, acide azélaïque et ses dérivés, un 4-méthyl-4-azastéroïde, en particulier la 17-$\beta$-N,N-diéthylcarbamoyl-4-méthyl-4-aza-5-$\alpha$ -androstan-3-one, ou encore un extrait de Serenoa repens.

Selon un troisième aspect, la présente invention couvre également l'utilisation, à titre d'ingrédient actif, de l'oxyacanthine, de l'un de ses sels d'addition d'acide, en particulier cosmétiquement ou pharmaceutiquement acceptables, ou d'un extrait de plante en contenant, à l'exclusion d'un extrait de la plante Berberis thunbergii, et de la plante Mahonia, pour la fabrication d'une composition pharmaceutique, notamment dermatologique, destinée à stimuler la pousse des cheveux, à retarder leur chute, ou à combattre le prurit, notamment le prurit du cuir chevelu ;
ou comme agent cosmétique destiné à stimuler la pousse des cheveux, à retarder leur chute, ou à combattre le prurit, notamment le prurit du cuir chevelu.

Selon un autre mode de réalisation, l'invention couvre également l'utilisation, à titre d'ingrédient actif, de l'oxyacanthine, de l'un de ses dérivés, de préférence de formule (I) précitée, de l'un de ses sels d'addition d'acide, en particulier cosmétiquement ou pharmaceutiquement acceptable, ou d'un extrait de plante en contenant, en association avec au moins une saponine, pour la fabrication d'une composition pharmaceutique, notamment dermatologique, destinée à stimuler la pousse des cheveux, à retarder leur chute, ou à combattre le prurit, notamment le prurit du cuir chevelu ;
ou comme agent cosmétique destiné à stimuler la pousse des cheveux, à retarder leur chute, ou à combattre le prurit, notamment le prurit du cuir chevelu.

Selon un autre mode de réalisation, l'invention couvre également l'utilisation d'une substance active constituée par l'oxyacanthine, l'un de ses dérivés, de préférence de formule (I) précitée, l'un de ses sels d'addition d'acide, en particulier cosmétiquement ou pharmaceutiquement acceptable, ou un extrait de plante en contenant, en présence de phases lamellaires lipidiques hydratées ou de liposomes, contenant ou non ladite substance active, pour la fabrication d'une composition pharmaceutique, notamment dermatologique, destinée à stimuler la pousse des cheveux, à retarder leur

chute, ou à combattre le prurit, notamment le prurit du cuir chevelu ;
ou comme agent cosmétique destiné à stimuler la pousse des cheveux, à retarder leur chute, ou à combattre le prurit, notamment le prurit du cuir chevelu.

Suivant un mode de réalisation particulier, le sel d'addition, l'extrait de plante et la saponine précités, de même que les concentrations pondérales des ingrédients actifs et leurs proportions relatives, sont tels que précédemment définis.

Les compositions cosmétique ou pharmaceutique, notamment dermatologique, conformément à la présente invention peuvent être appliquées par voie topique pour l'activité énoncée ci-dessus, en particulier dans des compositions se présentant sous forme de crème, de gel ou de lotion destinées à l'application sur le cuir chevelu.

Selon un quatrième aspect, la présente invention fournit également un procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'oxyacanthine, de l'un de ses dérivés, de préférence de formule (I) précitée, de l'un de ses sels d'addition d'acide ou d'un extrait de plante en contenant, à l'exclusion d'un extrait de la plante Berberis thunbergii, et de la plante Mahonia.

Selon un autre mode de réalisation, la présente invention fournit également un procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'une association d'oxyacanthine, de l'un de ses dérivés, de préférence de formule (I) précitée, de l'un de ses sels d'addition d'acide ou d'un extrait de plante en contenant, avec au moins une saponine.

Selon encore un autre mode de réalisation, la présente invention fournit un procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'une substance active constituée par l'oxyacanthine, l'un de ses dérivés, de préférence de formule (I) précitée, de l'un de ses sels d'addition d'acide ou d'un extrait de plante en contenant, en présence de phases lamellaires lipidiques hydratées ou de liposomes, contenant ou non ladite substance active.

Suivant un mode particulier de réaliation, le sel d'oxyacanthine et l'extrait de plante précités sont tels que précédemment définis.

Selon un autre mode de réalisation avantageux, l'oxyacanthine ci-dessus, l'un de ses sels d'addition d'acide ou l'extrait de plante en contenant, est associé à au moins une saponine telle que précédemment définie.

Selon un cinquième aspect, l'invention fournit encore un procédé de fabrication d'une composition cosmétique ou pharmaceutique, notamment dermatologique, destinée en particulier à stimuler la pousse des cheveux, à retarder leur chute, ou à combattre le prurit, notamment le prurit du cuir chevelu, caractérisé en ce qu'il comprend l'emploi d'oxyacanthine, de l'un de ses dérivés, de préférence de formule (I) précitée, de l'un de ses sels d'addition d'acide, en particulier un sel d'addition d'acide cosmétiquement ou pharmaceutiquement acceptable, ou d'un extrait de plante en contenant, que l'on mélange dans un excipient, véhicule ou support pharmaceutiquement ou cosmétiquement acceptable. Selon une variante de réalisation, l'oxyacanthine, ses dérivés ou l'un de ses sels d'addition d'acide précités ou l'extrait de plante en contenant, est associé avec au moins une saponine.

Dans l'un quelconque des aspects précédents de l'invention, la substance active de l'invention, à savoir l'oxyacanthine ou ses dérivés ou sels, ou un extrait de plante en contenant peut être utilisée en présence de phase lamellaires lipidiques hydratées ou de liposomes, incorporant ou non ladite substance active. Il est précisé que l'expression "incorporant" couvre le cas où la substance est totalement incorporée et celui où une certaine quantité seulement de cette substance est incorporée dans les phases lamellaires lipidiques hydratées ou dans les liposomes.

Le terme "lipidique" employé dans le présent document couvre toutes les substances comprenant une chaîne carbonée dite grasse, comportant généralement plus de 5 atomes de carbone, cette substance étant habituellement dénommée "lipide".

Selon l'invention, on utilise à titre de lipide, pour former soit les phases lamellaires lipidiques, soit les vésicules de type liposome, des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques ou des vésicules de type liposome en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja éventuellement hydrogénée, une phosphatidylcholine, une phosphatidylsérine, une sphyngomyéline, un cérébroside ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le β-sitostérol. La quantité, exprimée en mole, de lipide hydrophobe ne doit généralement pas être supérieure à la quantité de lipide amphiphile, et de préférence elle ne doit pas être supérieure à 0,5 fois cette quantité.

L'incorporation dans des phases lamellaires lipidiques hydratées ou dans des liposomes de la substance active

précitée utilisée conformément à la présente invention peut être réalisée selon des techniques de préparation connues, décrites par exemple dans le document US-A-4 508 703 et éventuellement en combinaison avec le document US-A-4 621 023.

Ainsi qu'il ressort des propriétés connues des liposomes, selon le caractère lipophile ou hydrophile de la substance à incorporer, celle est incorporée respectivement soit au moins en partie dans les bicouches de la phase lipidique des phases lamellaires lipidiques hydratées ou des liposomes, soit dans la phase aqueuse. Ainsi, l'oxyacanthine est incorporée dans ladite phase lipidique.

Dans l'un quelconque des aspects précédents de l'invention, la concentration minimale préférée en oxyacanthine, ses dérivés ou ses sels, dans l'extrait végétal est d'environ 0,001 %.

Egalement, dans l'un quelconque des aspects précédents de l'invention, un mode de réalisation avantageux concerne l'association d'oxyacanthine, ses dérivés ou sels, ou extrait en contenant, tel que l'extrait de B. vulgaris, avec le minoxidil ; de préférence l'oxyacanthine, ses dérivés ou sels ou les extraits en contenant étant incorporés en phase lamellaire lipidique hydratée ou en liposome, le tout étant mélangé dans un excipient compatible avec ces produits actifs, ou bien l'association est réalisée à partir des produits conditionnés séparément, soit sous forme d'un mélange effectué extemporanément, au moment de l'utilisation, soit par applications sensiblement simultanées ou successives de ces produits sur les zones à traiter.

D'autres buts, caractéristiques et avantages de l'invention apparaîtront clairement à la lecture de la description explicative qui va suivre, faite en référence à plusieurs exemples donnés uniquement à titre d'illustration et qui ne sauraient par conséquent en aucune façon limiter la portée de l'invention.

Dans les exemples, les pourcentages sont exprimés en poids, sauf indication contraire. Dans le cas des extraits, ceux-ci sont exprimés en poids sec de l'extrait.

Dans les figures annexées :

- La figure 1 rapporte des résultats d'essais comparatifs basés sur une étude trichocinétique relative au système pilaire du rat Sprague Dawley, avec une composition contenant 0,1 % d'un mélange de sulfates de bases tertiaires de Berberis vulgaris, incluant le sulfate d'oxyacanthine, relativement à un témoin excipient. En ordonnée, on a représenté le pourcentage de poils en phase anagène et en abscisse le nombre de jours ;
- La figure 2 est une figure similaire à la figure 1 mais réalisée avec une composition contenant 0,1 % du mélange de sulfates de bases tertiaires précité, associé à 0,1 % d'un mélange de saponines extrait de Medicago sativa ;
- La figure 3 représente des courbes similaires à celles de la figure 1, avec les résultats obtenus avec une composition comprenant 0,1 % de sulfate d'oxyacanthine du commerce associé à 0,1 % du mélange de saponines précité ;
- La figure 4 est une figure similaire à la figure 1 mais réalisée avec une composition contenant 0,1 % de sulfate d'oxyacanthine du commerce ;
- La figure 5 est une figure similaire à la figure 2 si ce n'est que les concentrations des constituants de la composition sont respectivement 0,18 % pour le mélange de sulfates de bases tertiaires et 0,09 % pour le mélange de saponines.
- La figure 6 est une figure similaire à la figure 1 si ce n'est que la comparaison concerne un extrait de Berberis à l'état libre, un extrait de Berberis encapsulé en liposome, des liposomes vides dits "blancs" relativement à un témoin ; et
- la figure 7 est une figure similaire à la figure 6 reprenant les courbes d'extrait de Berberis en liposome, d'extrait de Berberis à l'état libre en comparaison au minoxidil utilisé comme témoin "positif".

Exemple 1

Préparation d'un mélange de sulfates de bases tertiaires d'écorces de racines de Berberis vulgaris ou Epine-vinette

On broie 500 g d'écorces de racines séchées de Berberis vulgaris que l'on humidifie avec 300 ml environ d'ammoniaque 7N. On verse sur le tout 18 l d'acétate d'éthyle et on laisse macérer pendant 24 h. On filtre et on récupère d'une part le filtrat et d'autre part les marcs sur lesqueLs on effectue une lixiviation avec environ 30 l d'acétate d'éthyle. Le filtrat et le lixiviat sont réunis et on épuise cette phase organique par plusieurs quantités de solution aqueuse d'acide sulfurique à 2 %, jusqu'à ce que le test de Mayer, qui met en évidence la présence d'alcaloïdes, soit négatif sur les dernières eaux acides. On décante et on récupère la phase aqueuse que l'on alcalinise par de l'ammoniaque en présence de chloroforme. On décante à nouveau et on récupère d'une part la phase organique et d'autre part la phase aqueuse que l'on extrait au chloroforme, jusqu'à obtenir un test de Mayer pratiquement négatif sur les dernières eaux alcalines. Les phases chloroformiques sont lavées à l'eau, puis réunies et séchées sur du sulfate de sodium anhydre. On distille ensuite cette solution chloroformique, et on récupère ainsi 6,5 g d'un mélange de bases tertiaires contenant principalement de l'oxyacanthine (50 % environ) et de la berbamine. Ainsi, le rendement est d'environ 1,3 % par rapport à la matière sèche de départ.

Si on le souhaite, on peut séparer l'oxyacanthine des autres bases tertiaires, suivant une technique connue de

séparation, en particulier en se référant à la publication de A. ORECHOW, Archiv der Pharmazie (1933) 271, 323-7.

Pour la mise en oeuvre de la présente invention, on préfère utiliser le mélange de bases précité sous forme de mélange de sulfates que l'on obtient selon le procédé suivant.

Le mélange de bases tertiaires obtenu précédemment est dissous dans une quantité juste suffisante d'acétone. Cette solution est acidifiée à pH 3 avec de l'acide sulfurique concentré. Il se produit alors une précipitation des bases sous forme de sulfates. Après filtration, le précipité est dissous dans du méthanol, puis la solution est évaporée à sec. On obtient ainsi environ 6,7 g de mélange de sulfates de bases tertiaires incluant le sulfate d'oxyacanthine. Ce mélange sera désigné dans la suite de la description et des dessins par l'extrait "Extrait Sulfate" de Berberis vulgaris.

## Exemple 2

### Préparation d'un extrait d'écorces de racines de Berberis vulgaris par extraction alcoolique

On introduit 200 g d'écorces de racines séchées finement broyées de Berberis vulgaris dans un ballon, on ajoute environ 2 l de méthanol et on chauffe à reflux pendant 2 h. On laisse refroidir à température ambiante et on filtre. Le méthanol est ensuite distillé et on récupère ainsi environ 12 g d'extrait, ayant une teneur en oxyacanthine de 2 % environ.

De manière analogue, on peut obtenir des extraits avec d'autres parties de la plante, en particulier les tiges, et avec d'autres solvants tels que l'acétate d'éthyle ou un mélange hydro-alcoolique à 30 % en éthanol. Dans le cas de l'extraction avec de l'éthanol à 30 %, la solution contenant l'extrait est réduite par distillation à pression réduite, puis lyophilisée à -80°C. Les rendements moyens d'extraction par rapport à la matière sèche de départ et les taux d'oxyacanthine dans l'extrait sont rassemblés au tableau I ci-après.

TABLEAU I

| Parties de la plante | Rendement d'extraction | | | Taux d'oxyacanthine | | |
|---|---|---|---|---|---|---|
| | méthanol | éthanol 30 % | acétate d'éthyle | méthanol | éthanol 30 % | acétate d'éthyle |
| racines | 6 % | 10 % | | 2 % | 3-4 % | |
| tiges | 5 % | | < 1 % | 1 % | | 2 % |

## Exemple 3

### Préparation d'une composition selon l'invention comprenant un mélange de sulfates de bases tertiaires de Berberis vulgaris associé à un mélange de saponines de Medicago sativa

A - Préparation d'un mélange de saponines de racines de luzerne (Medicago sativa)

On prend 1 kg de poudre de racines de luzerne que l'on met à macérer dans 6 l de méthanol pendant 2 h. La suspension est portée au reflux pendant 3 h. Après refroidissement on filtre, et les marcs sont à nouveau soumis au traitement précédent avec du méthanol neuf. On répète l'opération 3 fois. Les phases méthanoliques sont réunies et évaporées sous vide. Le résidu est agité avec 200 ml d'éther éthylique. L'insoluble dans l'éther est récupéré par filtration et dissous dans 3 l d'eau qui sont extraits par du butanol normal saturé d'eau (3 l puis 2 x 2 l).

Les phases butanoliques sont réunies et évaporées sous vide.

Le résidu (environ 150 g) est dissous dans 1,5 l d'eau et dialysé contre de l'eau pure pendant 4 jours avec renouvellement quotidien de l'eau. Le contenu de la cellule dialysée est lyophilisé.

Avantageusement, on réalise une purification supplémentaire : le lyophilisat est dissous dans 300 ml de méthanol et jeté dans 1,5 l d'éther. Le précipité constitué du mélange de saponines est récupéré par filtration et séché une nuit sous vide, en présence de $P_2O_5$. Le rendement moyen est de 30 g.

B - Préparation de la composition selon l'invention

On dissout successivement 0,1 g de mélange de sulfates de bases tertiaires d'écorces de racines de Berberis préparé selon l'exemple 1, et 0,1 g du mélange de saponines de luzerne obtenu à l'étape ci-dessus, dans un excipient (EX) de composition : propylèneglycol 20 %, éthanol 63 %, eau 17 %.

On obtient ainsi une composition selon l'invention qui peut être utilisée telle quelle en application sur le cuir chevelu, en particulier pour retarder la chute des cheveux, stimuler leur pousse ou combattre les démangeaisons.

Exemple 4

Procédé d'obtention du chlorhydrate de méthyl-oxyacanthine

La méthylation a lieu sur l'oxyacanthine base.

A partir de 840 mg de sulfate, on extrait l'oxyacanthine de son sel par $CHCl_3$ (4 x 100 ml) en mi lieu alcalin (NaOH).

Après lavage à l'eau, séchage sur $Na_2SO_4$ et évaporation sous pression réduite à 40°C du $CHCl_3$, la base obtenue pèse environ 706 mg. Le rendement est de 97 %.

On procède à la méthylation de l'oxyacanthine base au moyen de diazométhane par dissolution de la base dans 30 ml de méthanol, refroidissement à 0°C, puis addition progressive de 20 ml de solution éthérée de diazométhane à 3 g/l.

La réaction, contrôlée par chromatographie en couche mince, est poursuivie 24 h à 0°C.

La méthylation est confirmée par RMN.

On prépare ensuite le chlorhydrate en concentrant la solution méthanolique du produit méthylé sous pression réduite jusqu'à obtenir environ 10 ml. On procède ensuite à l'addition de 1,1 ml de mélange méthanol/HCl 2N, puis on réalise la précipitation du sel de chlorhydrate par addition de 50 ml d'éther sec.

Les précipités obtenus après filtration sont lavés par l'acétone froide.

Après évaporation de l'acétone, on obtient 653 mg de sel de chlorhydrate qui ajoutés à la lyophilisation des eaux mères donnent 95 mg supplémentaires, soit au total 748 mg du sel de chlorhydrate de méthyl-oxyacanthine, ce qui donne un rendement de 92 %.

Exemple 5

Incorporation d'un mélange de sulfates de bases tertiaires d'écorces de racines de Berberis vulgaris dans des phases lamellaires lipidiques hydratées ou dans des liposomes

Un mélange de sulfates de bases tertiaires d'écorces de racines de Berberis vulgaris obtenu conformément à l'exemple 1 est incorporé dans des phases lamellaires lipidiques hydratées ou dans des liposomes selon la technique de préparation suivante :

on pèse :

- lécithine de soja : 18 g
- β-sitostéroL : 2 g
- mélange de sulfates de bases tertiaires de l'exemple 1 : 1 g.

Ces constituants sont dissous dans un mélange constitué de 300 ml de dichlorométhane.

On évapore le mélange ainsi obtenu dans un ballon rotatif à température de 45°C.

Le film lipidique ainsi obtenu est alors repris et dispersé sous agitation dans de l'eau distillée qsp 750 g, à température ambiante pendant 12 h.

La suspension de vésicules lipidiques obtenue est ensuite homogénéisée par un traitement aux ultrasons pendant 15 min à 4°C, sous puissance de 200 W, par fraction de 150 ml.

La taille moyenne des liposomes ainsi obtenus est de 134,4 ± 8,4 nanomètres.

Selon une variante de réalisation avantageuse, on gélifie ensuite cette suspension en la mélangeant à 250 g de gel de Carbopol® 980 à 1,25 % préparé séparément de façon classique.

On obtient ainsi 1 000 g environ d'une suspension gélifiée de liposomes encapsulant le mélange de sulfates de bases tertiaires,dont La concentration est d'environ 0,1 % par rapport au poids total de la suspension.

Ce gel est appelé "composition $I_6$" et peut être utilisé tel quel dans le cadre de l'invention.

Exemple 6

Mise en évidence de l'activité de l'oxyacanthine utilisée seule ou associée à une saponine sur le système pilaire

Le test repose sur l'étude de l'activité des produits selon l'invention sur le cycle pilaire de rats Sprague Dawley tous âgés de 23 j (j = jour). A cet âge, Les cycles pilaires de tous les animaux sont encore synchrones.

Plus particulièrement, on cherche à mettre en évidence l'activité de ces produits sur la prolongation de la phase de croissance des poils, dite "phase anagène".

On détermine en premier lieu le jour correspondant au nombre maximum de poils en phase anagène pendant le deuxième cycle pilaire commençant entre le 32e et le 35e j de vie des animaux.

Pour ce la, on opère de la façon suivante. Les rats sont tondus à 24 j sur la partie inférieure du dos, au niveau des flancs et de manière à ne laisser qu'une faible longueur de poils, juste nécessaire pour permettre les épilations ultérieures. A partir du 28e j et à intervalle de temps sensiblement régulier (environ tous les 2 ou 3 j), on prélève à l'aide d'une pince à épiler une touffe de poils sur le côté gauche de l'animal au niveau du flanc. On observe à fort grossissement les bulbes de 10 poils pris au hasard dans cette touffe, et on compte le nombre de poils en phase anagène reconnaissables à la forme caractéristique du bulbe. On détermine ainsi le pourcentage de poils en phase anagène, en fonction du temps.

Pour chacune des trois séries d'expériences qui seront décrites plus loin, on réalise cette détermination sur un lot de 10 animaux. Pour ces trois lots, on a reporté au tableau II ci-après la moyenne des pourcentages de poils en phase anagène en fonction de l'âge exprimé en jours.

TABLEAU II

| âge (jours) | 28 | 30 | 32 | 35 | 37 | 39 | 42 | 44 | 46 | 49 | 51 |
|---|---|---|---|---|---|---|---|---|---|---|---|
| 1er lot | 0 | 0 | 0 | 0 | 15 | 100 | 100 | 94 | 6 | 0 | 0 |
| 2e lot | 0 | 0 | 0 | 0 | 27 | 98 | 85 | 80 | 39 | 0 | 0 |
| 3e lot | 0 | 0 | 0 | 86 | 89 | 93 | 84 | 71 | 28 | 0 | 0 |

Il ressort de ces résultats que la phase anagène atteint son maximum au 39e j, avec une valeur proche de 100 %. Ainsi, à ce jour, pratiquement tous les poils de tous les animaux sont en croissance. Ensuite, le nombre de poils en phase anagène diminue, pour atteindre la valeur zéro vers 49 j.

Pour mettre en évidence la prolongation de la phase anagène, il suffira alors d'observer le ralentissement de la diminution du nombre de poils en phase anagène à partir de ce 39e j de vie des animaux.

La présente étude concerne les produits suivants :

- produit $I_1$ : Sulfate d'oxyacanthine du commerce à 0,1 % dans l'excipient EX décrit à l'exemple 3B,
- produit $I_2$ : "Extrait sulfate" de Berberis vulgaris selon l'exemple 1 à 0,1 % dans l'excipient EX,
- produit $I_3$ : Sulfate d'oxyacanthine du commerce à 0,1 % et mélange de saponines de racines de luzerne selon l'exemple 3A à 0,1 %, dans l'excipient EX,
- produit $I_4$ : Composition selon l'exemple 3B ("Extrait sulfate" de B. vulgaris 0,1 %, mélange de saponines de luzerne selon l'exemple 3A 0,1 % dans l'excipient EX),
- produit $I_5$ : Composition analogue à celle de l'exemple 3B, si ce n'est que les concentrations sont respectivement 0,18 % pour l'"Extrait sulfate" et 0,09 % pour le mélange de saponines. La composition de l'excipient est identique,
- produit EX : Excipient EX tel que décrit à l'exemple 3B.

Les produits $I_1$ à $I_5$ sont des produits selon l'invention, le produit EX est un produit témoin.

Trois séries d'expériences ont été effectuées pour tester les produits précités.

Les animaux sont tondus à 24 j, comme indiqué plus haut. On applique les produits à tester, sur la partie du dos qui a été tondue, à partir du 25e j et jusqu'au 65e j, 6 j sur 7, à une dose évoluant avec le poids des animaux. Cette dose est de 0,5 ml au 25e j, et atteint 2 ml au 65e j.

Dans chacune de ces séries d'expériences, on détermine, comme plus haut, le pourcentage de poils en phase anagène.

La série 1 est conduite sur 40 animaux répartis en 4 lots de 10 animaux. Le premier lot, qui reçoit le produit EX, est désigné par l'expression "témoin excipient". Les 2e, 3e et 4e lots reçoivent respectivement les produits $I_2$, $I_3$ et $I_4$. Les résultats figurent au tableau III et aux figures 1, 3 et 2.

La série 2 est conduite sur 20 animaux répartis en 2 lots de 10 animaux. Le premier lot est le lot "témoin excipient". Le deuxième lot reçoit le produit $I_1$. Les résultats figurent au tableau IV et à la figure 4.

La série 3 est également conduite sur 20 animaux répartis sur 2 lots : un lot "témoin excipient" et un lot recevant le produit $I_5$. Les résultats figurent au tableau V et à la figure 5.

Les résultats de cette étude trichocinétique (trichogramme en fonction du temps) sont, en valeurs moyennes, représentés sur les figures 1 à 5 annexées sur lesquelles on a représenté en ordonnée les pourcentages de poils en phase anagène, et en abscisse le temps, entre le 39e jour et le 51e jour de vie des animaux. Les tableaux III à V, ci-dessous, reportent les valeurs de surface obtenues sous les courbes entre le 39e j et le 51e j (S). Pour chaque valeur

de surface, on détermine l'activité mesurée par rapport à l'excipient servant de témoin de référence. En effet, on comprend que, sur une période donnée, la surface comprise entre une courbe et l'axe des abscisses est proportionelle à la quantité de poils en phase anagène. Ainsi, la détermination de cette surface sous chaque courbe permet de comparer quantitativement l'activité des produits, quant à l'augmentation des taux de poils en phase anagène, les uns par rapport aux autres et/ou par rapport aux témoins.

Le maximum du taux de poils en phase anagène chez le lot "témoin absolu" se situant vers le 39e jour, la détermination de la surface sous la courbe à partir de cette date permet d'apprécier pour chaque produit son effet sur la prolongation de la phase anagène, notamment par rapport aux témoins.

ACTIVITE DES PRODUITS DE L'INVENTION

Comparaison des surfaces de courbes

S : surface (exprimée en % x jour) sous la courbe correspondant aux produits $I_1$ à $I_5$ ou à l'excipient EX entre le 39e jour et le 51e jour.
$T_e$ : témoin excipient EX
$P_a$ : pourcentage de prolongation de la phase anagène (par rapport au témoin excipient)

$$P_a = \frac{S_I - S_{EX}}{S_{EX}} \times 100$$

TABLEAU III

| | $T_e$ | $I_2$ (figure 1) | $I_4$ (figure 2) | $I_3$ (figure 3) |
|---|---|---|---|---|
| S | 620,5 | 638 | 667,5 | 677,5 |
| $P_a$ % | 0 | 2,8 | 7,6 | 9,2 |

TABLEAU IV

| | $T_e$ | $I_1$ (figure 4) |
|---|---|---|
| S | 639 | 657 |
| $P_a$ % | 0 | + 2,8 |

TABLEAU V

| | $T_e$ | $I_5$ (figure 5) |
|---|---|---|
| S | 563 | 625,5 |
| $P_a$ % | 0 | 11,1 |

Sur les figures 1 à 5 annexées, les courbes joignant les cercles blancs correspondent aux résultats obtenus avec l'excipient témoin. A la figure 1, la courbe avec les triangles avec la pointe en bas correspond à l'emploi de la préparation $I_2$ ne contenant que l'"Extrait sulfate" de Berberis vulgaris à 0,1 % dans l'excipient. A la figure 2, la courbe avec les triangles ayant la pointe tournée vers le haut est relative à la préparation $I_4$ contenant l'"Extrait sulfate" de B. vulgaris à 0,1 % associé aux saponines de racines de luzerne selon l'exemple 3A à 0,1 % dans l'excipient. A la figure 3, la courbe reliant les croix est obtenue avec le produit $I_3$ contenant du sulfate d'oxyacanthine du commerce à 0,1 % associé aux saponines de racines de luzerne à 0,1 % dans l'excipient. A la figure 4, la courbe avec les losanges correspond à l'emploi de la préparation $I_1$ ne contenant que du sulfate d'oxyacanthine du commerce à 0,1 % dans l'excipient. Enfin,

à la figure 5, la courbe reliant les carrés est relative à la composition $I_5$ contenant l'"Extrait sulfate" de Berberis vulgaris à 0,18 % associé aux saponines de racines de luzerne selon l'exemple 3A à 0,09 %.

A partir des tableaux III à V, on observe que, par rapport aux témoins, la surface S représentant le taux de poils en phase anagène (phase de croissance), augmente sensiblement avec les produits de l'invention contenant l'"Extrait sulfate" de Berberis vulgaris incluant le sulfate d'oxyacanthine, ce phénomène étant d'autant plus net lorsque cet extrait ou le sulfate d'oxyacanthine est associé aux saponines de luzerne. En outre, on voit nettement sur les figures 1 à 5 que tous les produits de l'invention testés proLongent la phase de pousse des poils (phase anagène), puisqu'on observe dans le temps un pourcentage de poils anagènes plus élevé que chez les animaux témoins, donc un ralentissement de la diminution du nombre de poils anagènes, et un pourcentage non nul pendant plus longtemps.

Ainsi, il est clair que ces essais démontrent que l'oxyacanthine seule ou combinée avec au moins une saponine, par l'augmentation de la durée de la phase anagène, retarde très nettement la chute des cheveux, ce qui constitue une propriété particulièrement inattendue.

Exemple 7

Mise en évidence de l'activité d'un mélange de sulfates de bases tertiaires d'écorces de racines de Berberis vulgaris incorporé en liposomes, en comparaison notamment avec le minoxidil

On procède selon le même protocole qu'à l'exemple précédent, sur des rats Sprague Dawley tous âgés de 23 j (j = jour).

Toutefois, dans la présente expérimentation, sans doute en raison de l'époque différente de l'année à laquelle celle-ci a été effectuée, le jour correspondant au nombre maximum de poils en phase anagène est le 42e j.

La présente étude concerne les produits suivants :

- produit $I_2$ : "extrait sulfate" de Berberis vulgaris selon l'exemple 1 à 0,1 % dans l'excipient EX précité,
- produit $I_6$ : gel contenant l'"extrait sulfate" de Berberis vulgaris incorporé en liposomes selon l'exemple 5,
- produit L comparatif : gel liposomal préparé selon l'exemple 5, si ce n'est qu'il ne contient aucune substance active selon l'invention,
- produit M comparatif : minoxidil (témoin positif) à 2 % dans l'excipient EX précité.

L'étude est conduite sur 50 animaux répartis en 5 lots de 10 animaux.

Le premier et le deuxième lot reçoivent respectivement les produits $I_2$ et $I_6$ selon l'invention. Le troisième lot reçoit le produit L, le quatrième le produit M et le cinquième, lot témoin T, ne reçoit aucun produit. Les résultats figurent au tableau VI et aux figures 6 et 7, suivant une présentation analogue à celle des résultats de l'étude de l'exemple précédent.

ACTIVITE DES PRODUITS DE L'INVENTION

Comparaison des surfaces de courbes

S : surface (exprimée en % x jour) sous la courbe correspondant aux produits $I_2$ et $I_6$, L, M et à celle du lot témoin T, du 42e au 53e jour.

$P_{aT}$ : pourcentage de prolongation de la durée de la phase anagène (par rapport au témoin T)

$$P_{aT} = \frac{S_I - S_T}{S_T} \times 100$$

$A_M$ : activité sur la prolongation de la durée de la phase anagène - par rapport au minoxidil, témoin positif.

$$A_M = \frac{S_I}{S_M} \times 100$$

TABLEAU VI

| | T lot témoin | L | $I_2$ | $I_6$ | M |
|---|---|---|---|---|---|
| S | 625,5 | 643,5 | 636 | 674,5 | 520 |
| $P_{aT}$ | 0 | 3,20 | 1,68 | 7,83 | - 16,90 |
| $A_M$ | 120 | 124 | 122 | 130 | 100 |

Sur les figures annexées 6 et 7, les courbes joignant les triangles ayant la pointe tournée vers le bas sont relatives aux résultats du produit $I_2$, "Extrait sulfate" de Berberis vulgaris à 0,1 % dans l'excipient EX, celles reliant les triangles ayant leur pointe tournée vers le haut sont relatives aux résultats du produit $I_6$, gel de liposomes incorporant l'"Extrait sulfate" de Berberis vulgaris. Dans la figure 6, la courbe reliant les cercles blancs correspond aux résultats pour le lot témoin T, et celle reliant les ronds noirs correspond aux résultats du produit de comparaison L, gel liposomal précité. Dans la figure 7, la courbe reliant les croix correspond aux résultats pour le produit de comparaison M, minoxidil à 2 % dans l'excipient EX.

A partir du tableau VI, on observe que, par rapport au lot témoin, la phase anagène est prolongée sensiblement non seulement dans les lots ayant reçu les produits de l'invention $I_2$ et $I_6$, mais également dans celui ayant reçu le produit L, gel liposomal (liposomes vides dits "blancs"). Ce dernier phénomène observé est sans doute dû à l'activité intrinsèque des liposomes préparés selon le procédé décrit plus haut, au niveau des follicules pileux, sur la croissance du poil. Par contre, il apparait clairement une synergie entre les liposomes et l'"Extrait sulfate" lorsque celui-ci est incorporé, car le pourcentage de prolongation de la phase anagène $P_{aT}$ pour le produit $I_6$ est très nettement supérieur à la somme de ceux correspondant respectivement au produit L et au produit $I_2$.

Par ailleurs, les résultats obtenus pour l'activité $A_M$ par rapport au minoxidil, montrent que l'activité des produits de l'invention est très nettement supérieure. Il semble en effet que le minoxidil ne présente aucune action sur la proLongation de la phase anagène, bien au contraire. Toutefois, les résultats de notre étude sur la période précédant le 42e j, non rapportés ici, montrent une activité très supérieure du minoxidil au début de cette phase, c'est-à-dire une activité importante sur la stimulation de la repousse des cheveux.

Ainsi, il est clair que ces essais démontrent d'une part que les extraits de plante contenant de l'oxyacanthine, incorporés dans des liposomes, par une augmentation de la durée de la phase anagène plus importante que dans le cas où ces extraits ne sont pas incorporés, présentent une activité améliorée pour retarder la chute des cheveux, et d'autre part que les produits de la présente invention possèdent une activité supérieure à celle du minoxidil, dans cette application, ce qui constitue un avantage particulièrement inattendu.

On donnera ci-après divers exemples de formulation de compositions cosmétiques.

Exemple 8

Lotion capillaire retardant la chute des cheveux

| | |
|---|---|
| extrait hydro-éthanolique 30 % d'écorces de racines de Berberis vulgaris selon l'exemple 2 | 0,05 g |
| saponines de racines de luzerne selon l'exemple 3A | 0,05 g |
| Ceraphyl 60® | 0,10 g |
| éthanol absolu | 25,00 g |
| Phenonip® | 0,40 g |
| Cremophor RH40® | 0,50 g |
| excipient aqueux parfumé | qsp 100,00 g |

Exemple 9

Lotion capillaire tonique anti-chute

| | |
|---|---|
| sulfate d'oxyacanthine | 0,10 g |
| saponines de racines de luzerne selon l'exemple 3A | 0,10 g |
| BHA | 0,05 g |
| Ceraphyl 60® | 0,09 g |
| Cremophor RH40® | 0,50 g |
| éthanol | 32,00 g |
| eau + parfum | qsp 100,00 g |

Exemple 10

Lotion stimulante capillaire coiffante anti-chute

| | |
|---|---|
| "Extrait sulfate" de Berberis vulgaris selon l'exemple 1 | 0,20 g |
| saponines de racines de luzerne selon l'exemple 3A | 0,20 g |
| Ceraphyl 60® | 0,10 g |
| éthanol | 40,00 g |
| BHA | 0,01 g |
| propylèneglycol | 4,00 g |
| Cremophor RH40® | 0,50 g |
| eau + parfum | qsp 100,00 g |

Exemple 11

Gel traitant capillaire prolongeant la phase de croissance des cheveux

| | |
|---|---|
| "Extrait sulfate" de Berberis vulgaris selon l'exemple 1 | 0,05 g |
| saponines de racines de luzerne selon l'exemple 3A | 0,02 g |
| éthanol | 30,00 g |
| Cremophor RH40® | 0,50 g |
| gel de Carbopol 980® à 2 % | 50,00 g |
| eau | qsp 100,00 g |

Exemple 12

Lotion capillaire anti-prurit

| | |
|---|---|
| extrait hydro-éthanolique 30 % de tiges de Berberis vulgaris selon l'exemple 2 | 0,15 g |
| Ceraphyl 60® | 0,15 g |
| éthanol | 30,00 g |
| Phenonip® | 0,40 g |
| huile de ricin hydrogénée éthoxylée | 0,50 g |
| excipient aqueux parfumé | qsp 100,00 g |

Exemple 13

Emulsion calmante pour la peau

| | | |
|---|---|---|
| Phase A | Tefose 1500® | 7,00 g |
| | alcool cétylique | 2,00 g |
| | huile de paraffine | 20,00 g |
| | squalane | 5,00 g |
| Phase B | Cremophor RH40® | 0,50 g |
| | extrait hydro-éthanolique 30 % de tiges de B. vulgaris | 0,10 g |
| Phase C | gel de Carbopol 980® à 2,5 % | 12,00 g |
| Phase D | Germaben II® | 0,8 g |

On solubilise l'extrait de tiges de Berberis dans le Cremophor RH40® à chaud, on ajoute l'eau et on chauffe à 80°C.

A cette phase, on incorpore la phase A portée préalablement à 80°C. On homogénéise. On incorpore ensuite à 60°C la phase C sous homogénéisation, puis on laisse refroidir à 40°C et on incorpore la phase D. Enfin, on maintient l'homogénéisation de l'émulsion jusqu'à température ambiante pendant environ 1 h.

Exemple 14

Emulsion calmante pour la peau

| | | |
|---|---|---|
| Phase A | Cremophor A6® | 2,00 g |
| | Cremophor A25® | 2,00 g |
| | monostéarate de glycérol | 3,00 g |
| | alcool cétylique | 3,00 g |
| | huile de paraffine | 6,00 g |
| | huile d'amande douce | 6,00 g |
| | squalane | 2,20 g |
| Phase B | extrait hydro-éthanolique 30 % d'écorce de racine de B. vulgaris | 0,10 g |
| | p-hydroxybenzoate de méthyle | 0,15 g |
| | glycérine | 4,00 g |
| | eau | 71,55 g |

On chauffe séparément les phases A et B à 80°C.

Lorsque ces phases sont homogènes et à la même température, on incorpore la phase B dans la phase A sous agitation, que l'on maintient jusqu'à ce que l'émulsion revienne à la température ambiante, soit pendant environ 1 h à 1 h 30.

Exemple 15

Lotion capillaire anti-chute

| | |
|---|---|
| Chlorhydrate de méthyloxyacanthine préparé selon l'exemple 4 | 0,05 g |
| propylèneglycol | 10 g |
| alcool éthylique 95 % | 30 g |
| excipient aqueux parfumé qsp | 100 g |

Exemple 16

Composition dermo-cosmétique

"Duo" pour ralentir la chute des cheveux et stimuler leur repousse

Ce "duo" se présente sous la forme de deux produits A et B conditionnés séparément, dans deux récipients placés côte à côte, mais se mélangeant extemporanément au moment de l'emploi. Par exemple, on peut utiliser pour cela un dispositif basé sur le principe de celui décrit dans le document FR-A-2 603 558 = US-A-4773562, ou de celui commercialisé par la société VALOIS-France, sous le nom de DUOPACK®.

Produit A :

Gel liposomal incorporant un mélange de sulfates de bases tertiaires d'écorces de racines de Berberis vulgaris, selon l'exemple 5, dans lequel peuvent être rajoutés conservateurs et parfums..., 30 ml.

Produit B :

| | |
|---|---|
| 10 ml de la composition suivante : minoxidil | 8 % |
| propylèneglycol | 20 % |
| éthanol à 95 % | 63 % |
| excipient aqueux parfumé qsp 100 % | 10 ml |

Le dispositif est de préférence réglé par construction de manière à délivrer simultanément 3 volumes au moins de produit A pour un volume de produit B, de sorte que la concentration de l'excipient alcoolique de B ne soit pas telle que les liposomes de A soient déstabilisés.

Ce "duo" est utilisé de préférence en traitement d'attaque, à raison d'une application sur le cuir chevelu matin et soir pendant 3 mois.

**Revendications**

1. Composition cosmétique, destinée à stimuler la pousse des cheveux, à retarder leur chute, ou à combattre le prurit, notamment le prurit du cuir chevelu, caractérisée en ce qu'elle comprend, à titre d'ingrédient actif, une quantité cosmétiquement efficace d'oxyacanthine, ou de l'un de ses dérivés ou sels, de préférence de formule (I) suivante :

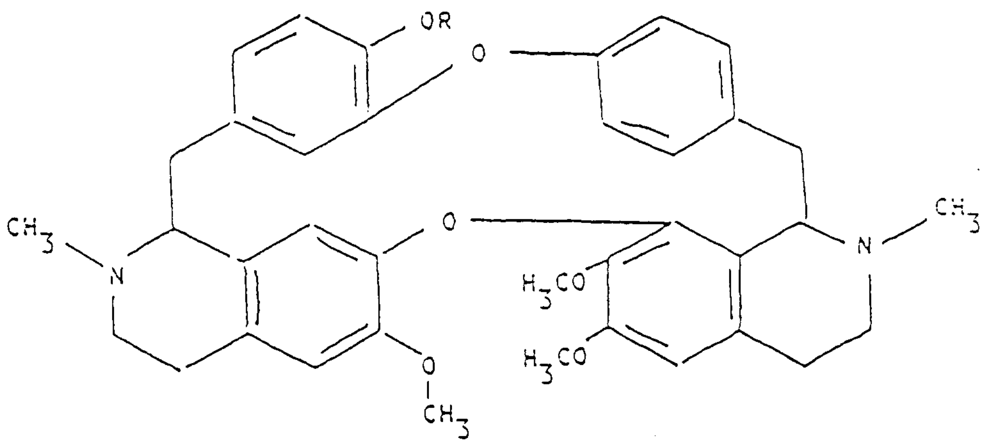

(I)

dans laquelle R représente l'hydrogène (oxyacanthine), une chaîne hydrocarbonée en $C_1$-$C_{12}$ ou un radical acyle en $C_2$-$C_{12}$,
la chaîne hydrocarbonée et le radical acyle peuvent être saturés ou non, linéaires, ramifiés ou en partie cycliques, notamment aromatiques ;

R représente en particulier un radical méthyle, hexyle, décyle, acétyle, propionyle, butanoyle, pentanoyle, hexanoyle, octanoyle, encore mieux méthyle, acétyle ou propionyle ;
d'un de ses sels d'addition d'acide, en particulier cosmétiquement acceptable ;
ou d'un extrait de plante contenant l'oxyacanthine ou ses dérivés ou sels précités, à l'exclusion d'un extrait de la plante Berberis thunbergii, et de la plante Mahonia.

2. Composition cosmétique ou pharmaceutique, notamment dermatologique, destinée à stimuler la pousse des cheveux, à retarder leur chute, ou à combattre le prurit, notamment le prurit du cuir chevelu, caractérisée en ce qu'elle comprend, à titre d'ingrédient actif, une quantité cosmétiquement ou pharmaceutiquement efficace d'une association constituée par l'oxyacanthine, l'un de ses dérivés ou sels, de préférence de formule (I) précitée ou l'un de ses sels d'addition d'acide, en particulier cosmétiquement ou pharmaceutiquement acceptable, ou d'un extrait de plante en contenant, et au moins une saponine.

3. Composition cosmétique ou pharmaceutique, notamment dermatologique, destinée à stimuler la pousse des cheveux, à retarder leur chute, ou à combattre le prurit, notamment le prurit du cuir chevelu, caractérisée en ce qu'elle

comprend, à titre d'ingrédient actif, une quantité cosmétiquement ou pharmaceutiquement efficace d'une substance active constituée par l'oxyacanthine, l'un de ses dérivés ou sels, de préférence de formule (I) précitée ou l'un de ses sels d'addition d'acide, en particulier cosmétiquement ou pharmaceutiquement acceptable, ou d'un extrait de plante en contenant, en présence de phases lamellaires lipidiques hydratées ou de liposomes, incorporant ou non ladite substance active.

**4.** Composition selon l'une des revendications 1 à 3, caractérisée en ce qu'elle contient du sulfate d'oxyacanthine ou un mélange de sulfates de bases tertiaires incluant le sulfate d'oxyacanthine, ce mélange étant généralement préparé à partir d'un extrait de plante.

**5.** Composition selon l'une quelconque des revendications 1 à 4, caractérisée en ce que l'extrait de plante précité est un extrait de plante choisie parmi les espèces suivantes : Berberis, en particulier B. amurensis Rupr., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib, B. laurina, B. nummularia, B. oblonga, B. orthobotrys, B. paucidentata Rusby, B. polyantha Hemsl., B. poiretii Schneid., B. pseudambalata, B. sargentiana Schneid., B. silvataroucana Schneid., B. soulieana Schneid., B. stolonifera, B. vernae Schneid., B. vulgaris ou épine vinette, B. wilsonae Hemsl. ; Mahonia, en particulier M. repens, M. aquifolia ; Laurelia, en particulier L. sempervirens ; Cocculus, en particulier C. laurifolius ; Xanthorhiza, en particulier X. simplicissima, les parties de plante utilisées pour la préparation dudit extrait étant les feuilles, les tiges, les racines, les baies, et de préférence l'écorce des tiges ou des racines.

**6.** Composition selon l'une quelconque des revendications 1 à 4, caractérisée en ce que l'extrait précité est un extrait de Berberis à l'exclusion de Berberis thunbergii, de préférence choisi parmi B. vulgaris, B. cretica, B. pseudambalata, B. stolonifera, B. laurina, en particulier un extrait d'écorce de racines de cette plante.

**7.** Composition selon l'une quelconque des revendications 1 à 6, caractérisée en ce que la concentration pondérale en oxyacanthine, en l'un de ses dérivés ou en l'un de ses sels d'addition d'acide précités, par rapport au poids total de la composition, est comprise entre 0,001 % et 5 %, de préférence entre 0,01 % et 2 %.

**8.** Composition selon l'une quelconque des revendications 2, 4 à 7, caractérisée en ce que la saponine précitée est choisie parmi le groupe des saponines stéroïdiques, notamment les saponines à sapogénine du type "diosgénine" et celles à sapogénine du type "sarsapogénine", et des saponines triterpéniques, notamment les saponines à sapogénine de structure "oléanane", telles que l'acide oléanolique, l'acide médicagénique, le sapogénol ou l'hédéragénine et celles à sapogénine de structure "ursane", telle que l'acide ursolique ou l'acide asiatique.

**9.** Composition selon l'une quelconque des revendications 2, 4 à 8, caractérisée en ce que la saponine précitée est extraite de l'une des plantes suivantes : Tribulus terrestris, Dioscorea, Smilax excelsa, Paris polyphylla, Cornus florida, Yucca, Smilax aristolochiaefolia, Asparagus officinalis, Hedera helix, Medicago - en particulier Medicago sativa ou luzerne -, Centella asiatica, Trigonella fenugraceum.

**10.** Composition selon la revendication 9, caractérisée en ce qu'elle contient un mélange de saponines extrait de l'une des plantes précitées, en particulier de Medicago sativa.

**11.** Composition selon l'une des revendications 2, 4 à 10, caractérisée en ce que les proportions relatives d'oxyacanthine ou de l'un de ses sels d'addition d'acide ou un extrait de plante en contenant et d'une saponine ou d'un mélange de saponines précités sont comprises entre 10/1 et 1/10 en poids, de préférence ce rapport est de 1/1 en poids.

**12.** Composition selon l'une des revendications 1 à 11, caractérisée en ce qu'elle comprend en outre au moins une autre substance active, à une concentration efficace, choisie parmi la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, des inhibiteurs de 5-$\alpha$-réductase tels que : progestérone, cyprotérone acétate, Minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

**13.** Composition selon l'une des revendications 1 à 12, caractérisée en ce qu'elle comprend une association d'oxyacanthine, ses dérivés ou ses sels, ou un extrait en contenant, tel qu'un extrait de B. vulgaris, avec le minoxidil, de préférence l'oxyacanthine, ses dérivés ou sels, ou les extraits en contenant étant incorporés en phases lamellaires lipidiques hydratées ou en liposomes, le tout étant mélangé dans un excipient compatible avec ces produits actifs,

ou bien l'association est réalisée à partir des produits conditionnés séparément, soit sous forme d'un mélange effectué extemporanément, au moment de l'utilisation, soit par application sensiblement simultanée ou successive de ces produits sur les zones à traiter.

**14.** Utilisation, à titre d'ingrédient actif, de l'oxyacanthine, de l'un de ses dérivés ou sels, de préférence de formule (I) précitée, de l'un de ses sels d'addition d'acide, cosmétiquement ou pharmaceutiquement acceptables, ou d'un extrait de plante en contenant, à l'exclusion d'un extrait de la plante Berberis thunbergii, et de la plante Mahonia, pour la fabrication d'une composition pharmaceutique, notamment dermatologique, destinée à stimuler la pousse des cheveux, à retarder leur chute ou à combattre le prurit, notamment le prurit du cuir chevelu ;
ou comme agent cosmétique destiné à stimuler la pousse des cheveux, à retarder leur chute ou à combattre le prurit, notamment le prurit du cuir chevelu.

**15.** Utilisation, à titre d'ingrédient actif, de l'oxyacanthine, de l'un de ses dérivés ou sels, de préférence de formule (I) précitée, de l'un de ses sels d'addition d'acide, cosmétiquement ou pharmaceutiquement acceptables, ou d'un extrait de plante en contenant, en association avec au moins une saponine, pour la fabrication d'une composition pharmaceutique, notamment dermatologique, destinée à stimuler la pousse des cheveux, à retarder leur chute ou à combattre le prurit, notamment le prurit du cuir chevelu ;
ou comme agent cosmétique destiné à stimuler la pousse des cheveux, à retarder leur chute ou à combattre le prurit, notamment le prurit du cuir chevelu.

**16.** Utilisation d'une substance active constituée par l'oxyacanthine, l'un de ses dérivés ou sels, de préférence de formule (I) précitée, l'un de ses sels d'addition d'acide, cosmétiquement ou pharmaceutiquement acceptables, ou un extrait de plante en contenant, en présence de phases lamellaires lipidiques hydratées ou de liposomes, incorporant ou non ladite substance active, pour la fabrication d'une composition pharmaceutique, notamment dermatologique, destinée à stimuler la pousse des cheveux, à retarder leur chute ou à combattre le prurit, notamment le prurit du cuir chevelu ;
ou comme agent cosmétique destiné à stimuler la pousse des cheveux, à retarder leur chute ou à combattre le prurit, notamment le prurit du cuir chevelu.

**17.** Utilisation selon l'une des revendications 14 à 16, caractérisée en ce que l'ingrédient actif est le sulfate d'oxyacanthine ou un mélange de sulfates de bases tertiaires incluant le sulfate d'oxyacanthine, ce mélange étant généralement préparé à partir d'un extrait de plante.

**18.** Utilisation selon l'une des revendications 14 à 17, caractérisée en ce que l'extrait de plante précité est un extrait de plante choisie parmi les espèces suivantes : Berberis, en particulier B. amurensis Rupr., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib, B. laurina, B. nummularia, B. oblonga, B. orthobotrys, B. paucidentata Rusby, B. polyantha Hemsl., B. poiretii Schneid., B. pseudambalata, B. sargentiana Schneid., B. silvataroucana Schneid., B. soulieana Schneid., B. stolonifera, B. vernae Schneid., B. vulgaris ou épine vinette, B. wilsonae Hemsl. ; Mahonia, en particulier M. repens, M. aquifolia ; Laurelia, en particulier L. sempervirens ; Cocculus, en particulier C. laurifolius ; Xanthorhiza, en particulier X. simplicissima, les parties de plante utilisées pour la préparation dudit extrait étant les feuilles, les tiges, les racines, les baies, et de préférence l'écorce des tiges ou des racines.

**19.** Utilisation selon l'une des revendications 14 à 18, caractérisé en ce que l'extrait précité est un extrait de Berberis à l'exclusion de Berberis thunbergii, de préférence choisi parmi B. vulgaris, B; cretica, B. pseudambalata, B. stolonifera, B. laurina, en particulier un extrait d'écorce de racines de cette plante.

**20.** Utilisation selon l'une quelconque des revendications 14 à 19, caractérisée en ce que la concentration pondérale en oxyacanthine ou en l'un de ses sels d'addition d'acide précités, par rapport au poids total de la composition, est comprise entre 0,001 % et 5 %, de préférence entre 0,01 % et 2 %.

**21.** Utilisation selon l'une quelconque des revendications 14 à 20, caractérisée en ce que la saponine précitée est choisie parmi le groupe des saponines stéroïdiques, notamment les saponines à sapogénine du type "diosgénine" et celles à sapogénine du type "sarsapogénine", et des saponines triterpéniques, notamment les saponines à sapogénine de structure "oléanane", telles que l'acide oléanolique, l'acide médicagénique, le sapogénol ou l'hédéragénine et celles à sapogénine de structure "ursane", telles que l'acide ursolique ou l'acide asiatique.

**22.** Utilisation selon l'une quelconque des revendications 14 à 21, caractérisée en ce que la saponine précitée est extraite de l'une des plantes suivantes : Tribulus terrestris, Dioscorea, Smilax excelsa, Paris polyphylla, Cornus florida, Yucca, Smilax aristolochiaefolia, Asparagus officinalis, Hedera helix, Medicago - en particulier Medicago sativa ou luzerne -, Centella asiatica, Trigonella fenugraecum.

**23.** Utilisation selon la revendication 22, caractérisée en ce qu'elle porte sur un mélange de saponines extrait de l'une des plantes précitées, en particulier de Medicago sativa.

**24.** Utilisation selon l'une des revendications 14 à 23, caractérisée en ce que les proportions relatives d'oxyacanthine ou de l'un de ses sels d'additions d'acide ou un extrait de plante en contenant et d'une saponine ou d'un mélange de saponines précités sont comprises entre 10/1 et 1/10 en poids, de préférence ce rapport est de 1/1 en poids.

**25.** Utilisation selon l'une des revendications 14 à 24, caractérisée en ce que la composition précitée comprend en outre au moins une autre substance active, à une concentration efficace, choisie parmi la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, des inhibiteurs de 5-$\alpha$-réductase tels que : progestérone, cyprotérone acétate, Minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

**26.** Utilisation selon l'une des revendications 14 à 25, caractérisée en ce que la composition précitée comprend l'association d'oxyacanthine, de l'un de ses dérivés ou sels, ou un extrait en contenant, tel qu'un extrait de B. vulgaris, avec le Minoxidil ; de préférence l'oxyacanthine, ses dérivés ou sels, ou les extraits en contenant, sont incorporés en phases lamellaires lipidiques hydratées ou en liposomes, le tout étant mélangé dans un excipient compatible avec ces produits actifs, ou bien l'association est réalisée à partir des produits conditionnés séparément, soit sous forme d'un mélange effectué extemporanément, au moment de l'utilisation, soit par applications sensiblement simultanées ou successives de ces produits sur les zones à traiter.

**27.** Procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'oxyacanthine, de l'un de ses dérivés ou sels, de formule (I) précitée, de l'un de ses sels d'addition d'acide ou d'un extrait de plante en contenant, à l'exclusion d'un extrait de Berberis thunbergii, et de Mahonia.

**28.** Procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'oxyacanthine, de l'un de ses dérivés ou sels, de formule (I) précitée, de l'un de ses sels d'addition d'acide ou d'un extrait de plante en contenant, en association avec au moins une saponine.

**29.** Procédé de traitement cosmétique destiné à favoriser la pousse des cheveux, à retarder leur chute ou à combattre le prurit, notamment le prurit du cuir chevelu, caractérisé en ce qu'il comprend l'application sur la zone à traiter, d'une quantité efficace, pour réaliser ledit effet recherché, d'une substance active constituée par l'oxyacanthine, l'un de ses dérivés ou sels, de formule (I) précitée, de l'un de ses sels d'addition d'acide ou un extrait de plante en contenant, en présence de phases lamellaires lipidiques hydratées ou de liposomes contenant ladite substance active.

**30.** Procédé selon l'une des revendications 27 à 29, caractérisé en ce que l'oxyacanthine précitée, l'un de ses dérivés, l'un de leurs sels d'addition d'acide ou l'extrait de plante en contenant, est associé à au moins une saponine.

**31.** Procédé selon l'une quelconque des revendications 27 à 30, caractérisé en ce que l'oxyacanthine précitée, l'un de ses dérivés, l'un de leurs sels d'addition d'acide ou l'extrait de plante en contenant, est incorporé dans des phases lamellaires lipidiques hydratées ou des liposomes.

**32.** Procédé selon l'une quelconque des revendications 27 à 31, caractérisé en ce qu'on applique une composition contenant de 0,001 % à 5 % en poids d'oxyacanthine, de l'un de ses dérivés ou sels, de préférence de formule (I) précitée ou d'un extrait de plante en contenant par rapport au poids total de la composition.

**33.** Procédé selon l'une quelconque des revendications 27 à 32, caractérisé en ce qu'on associe l'oxyacanthine, l'un dc ses dérivés ou sels, ou un extrait de plante en contenant, tel qu'un extrait de B. vulgaris, avec le minoxidil, de préférence l'oxyacanthine, ses dérivés ou sels, ou les extraits en contenant, est incorporé en phases lamellaires lipidiques hydratées ou en liposomes, le tout étant mélangé dans un excipient compatible avec ces produits actifs,

ou bien l'association est réalisée à partir des produits conditionnés séparément, soit sous forme d'un mélange effectué extemporanément au moment de l'utilisation, soit par applications sensiblement simultanées ou successives de ces produits sur les zones à traiter.

## Claims

1. A cosmetic composition, intended for stimulating hair growth, retarding hair loss or combating pruritus, especially pruritus of the scalp, characterized in that it comprises, as active ingredient, a cosmetically effective amount of oxyacanthine or one of its derivatives or salts, preferably of formula (I) below:

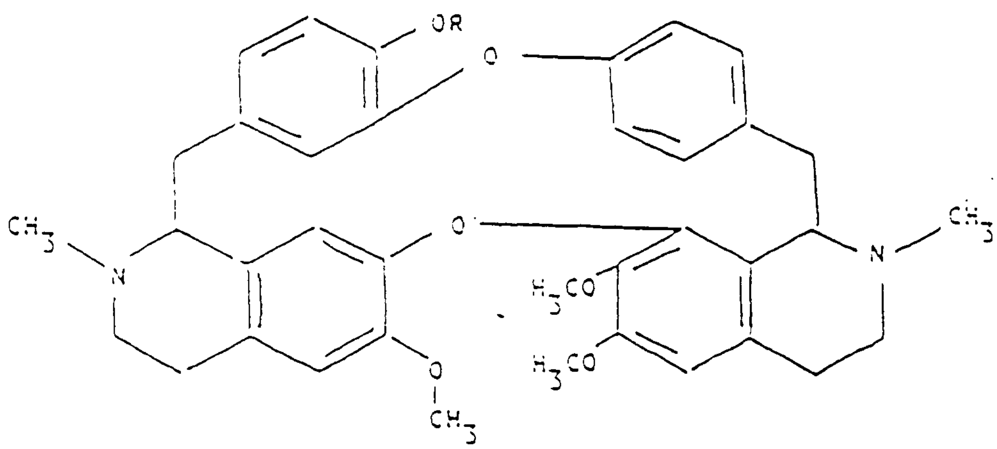

in which R is hydrogen (oxyacanthine), a $C_1$-$C_{12}$ hydrocarbon chain or a $C_2$-$C_{12}$ acyl radical,
the hydrocarbon chain and the acyl radical can be saturated or unsaturated, linear, branched or partly cyclic, especially aromatic;

R is in particular a methyl, hexyl, decyl, acetyl, propionyl, butanoyl, pentanoyl, hexanoyl or octanoyl radical, preferably a methyl, acetyl or propionyl radical;
one of its acid addition salts, in particular one which is cosmetically acceptable;
or a plant extract containing oxyacanthine or its above-mentioned salts or derivatives, with the exclusion of an extract of the plant Berberis thunbergii and of the plant Mahonia

2. A cosmetic or pharmaceutical composition, especially dermatological composition, intended for stimulating hair growth, retarding hair loss or combating pruritus, especially pruritus of the scalp, characterized in that it comprises, as active ingredient, a cosmetically or pharmaceutically effective amount of an association consisting of oxyacanthine, one of its derivatives or salts, preferably of formula (I) given above, one of its acid addition salts, in particular one which is cosmetically or pharmaceutically acceptable, or a plant extract in which it is present, with at least one saponin.

3. A cosmetic or pharmaceutical composition, especially dermatological composition, intended for stimulating hair growth, retarding hair loss or combating pruritus, especially pruritus of the scalp, characterized in that it comprises, as active ingredient, a cosmetically or pharmaceutically effective amount of an active substance consisting of oxyacanthine, one of its derivatives or salts, preferably of formula (I) given above, one of its acid addition salts, in particular one which is cosmetically or pharmaceutically acceptable, or a plant extract in which it is present, in the presence of hydrated lipidic lamellar phases or of liposomes, which may or may not incorporate said active substance.

4. The composition according to one of claims 1 to 3, characterized in that it contains oxyacanthine sulfate or a mixture of sulfates of tertiary bases including oxyacanthine sulfate, this mixture generally being prepared from a plant extract.

5. The composition according to any one of claims 1 to 4, characterized in that the above-mentioned plant extract is an extract of a plant selected from the following species: Berberis, in particular B. amurensis Ruper., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib., B. laurina, B. nummularia, B. oblonga, B. orthobotrys, B. paucidentata Rusby, B. polyantha Hemsl., B. poiretii Schneid., B. pseudambalata, B. sargentiana Schneid., B. silvataroucana Schneid., B. soulieana Schneid., B. stolonifera, B. vernae Schneid., B. vulgaris, or barberry, and B. wilsonae Hemsl.; Mahonia, in particular M. repens and M. aquifolia; Laurelia, in particular L. sempervirens; Cocculus, in particular C. laurifolius; and Xanthorhiza, in particular X. simplicissima, the parts of the plant used for the preparation of said extract being the leaves, the stems, the roots and the berries and preferably the cortex of the stems or roots.

**6.** The composition according to any one of claims 1 to 4, characterized in that the above-mentioned extract is an extract of Berberis with the exclusion of Berberis thunbergii, preferably selected from B. vulgaris, B. cretica, B. pseudambalata, B. stolonifera and B. laurina, in particular an extract of the root cortex of this plant.

**7.** The composition according to any one of claims 1 to 6, characterized in that the concentration by weight of oxyacanthine, one of its derivatives or one of its acid addition salts, as mentioned above, is between 0.001% and 5% , preferably between 0.01% and 2%, based on the total weight of the composition.

**8.** The composition according to any one of claims 2, 4 to 7, characterized in that the above-mentioned saponin is selected from the group comprising the steroid saponins, especially the saponins containing sapogenin of the "diosgenin" type and those containing sapogenin of the "sarsapogenin" type, and the triterpene saponins, especially the saponins containing sapogenin of the "oleanane" structure, such as oleanolic acid, medicagenic acid, sapogenol or hederagenin, and those containing sapogenin of the "ursane" structure, such as ursolic acid or asiatic acid.

**9.** The composition according to any one of claims 2, 4 to 8, characterized in that the above-mentioned saponin is extracted from one of the following plants: Tribulus terrestris, Dioscorea, Smilax excelsa, Paris polyphylla, Cornus florida, Yucca, Smilax aristolochiaefolia, Asparagus officinalis, Hedera helix, Medicago - in particular Medicago sativa or lucerne - Centella asiatica and Trigonella fenugraecum.

**10.** The composition according to claim 9, characterized in that it contains a mixture of saponins extracted from one of the above-mentioned plants, in particular from Medicago sativa.

**11.** The composition according to one of claims 2, 4 to 10, characterized in that the relative proportions of oxyacanthine, one of its acid addition salts or a plant extract in which it is present, and of a saponin or a mixture of saponins, as mentioned above, are between 10/1 and 1/10 by weight, this ratio preferably is 1/1 by weight.

**12.** The composition according to one of claims 1 to 11, characterized in that it also comprises an effective concentration of at least one other active substance selected from quinine or its derivatives, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone, acetate, minoxidil, azelaic acid and its derivatives, or a 4-methyl-4-azasteroid, in particular 17-$\beta$-N,N-diethylcarbamoyl-4-methyl-4-aza-5-$\alpha$ -androstan-3-one, or else an extract of Serenoa repens.

**13.** The composition according to one of claims 1 to 12, characterized in that it comprises an association of oxyacanthine, its derivatives or salts or an extract in which it is present, such as an extract of B. vulgaris, with minoxidil, the oxyacanthine, its derivatives or salts where the extracts in which it is present preferably are incorporated into hydrated lipidic lamellar phases or into liposomes, the whole being mixed with an excipient compatible with these active products, or else the association is produced from the separately packaged products, either in the form of a mixture prepared immediately before use, or by substantially simultaneous or successive application of these products to the areas to be treated.

**14.** Use, as an active ingredient, of oxyacanthine, one of its derivatives or salts, preferably of formula (I) given above, one of its acid addition salts, those which are cosmetically or pharmaceutically acceptable, or a plant extract in which it is present, with the exclusion of an extract of the plant Berberis thunbergii and of the plant Mahonia, for the manufacture of a pharmaceutical composition, especially dermatological composition, intended for stimulating hair growth, retarding hair loss or combating pruritus, especially pruritus of the scalp;
or as a cosmetic agent intended for stimulating hair growth, retarding hair loss or combating pruritus, especially pruritus of the scalp.

**15.** The use, as an active ingredient, of oxyacanthine, one of its derivatives or salts, preferably of formula (I) given above, one of its acid addition salts, those which are cosmetically or pharmaceutically acceptable, or a plant extract

in which it is present, in association with at least one saponin, for the manufacture of a pharmaceutical composition, especially dermatological composition, intended for stimulating hair growth, retarding hair loss or combating pruritus, especially pruritus of the scalp;
or as a cosmetic agent intended for stimulating hair growth, retarding hair loss or combating pruritus, especially pruritus of the scalp.

**16.** The use of an active substance consisting of oxyacanthine, one of its derivatives or salts, preferably of formula (I) given above, one of its acid addition salts, those which are cosmetically or pharmaceutically acceptable, or a plant extract in which it is present, in the presence of hydrated lipidic lamellar phases or of liposomes, which may or may not incorporate said active substance, for the manufacture of a pharmaceutical composition, especially dermatological composition, intended for stimulating hair growth, retarding hair loss or combating pruritus, especially pruritus of the scalp;
or as a cosmetic agent intended for stimulating hair growth, retarding hair loss or combating pruritus, especially pruritus of the scalp.

**17.** The use according to one of claims 14 to 16, characterized in that the active ingredient is oxyacanthine sulfate or a mixture of sulfates of tertiary bases including oxyacanthine sulfate, this mixture generally being prepared from a plant extract.

**18.** The use according to one of claims 14 to 17, characterized in that the above-mentioned plant extract is an extract of a plant selected from the following species: Berberis, in particular B. amurensis Ruper., B. boliviana Lechl., B. bumeliaefolia Schneid., B. buxifolia, B. chingi Cheng., B. coriaria, B. cretica, B. dielsiana Fedde., B. integerrima, B. julianea Schneid., B. koreana Palib., B. laurina, B. nummularia, B. oblonga, B. orthobotrys, B. paucidentata Rusby, B. polyantha Hemsl., B. poiretii Schneid., B. pseudambalata, B. sargentiana Schneid., B. silva-taroucana Schneid., B. soulinea Schneid., B. stolonifera, B. vernae Schneid., B. vulgaris, or barberry, and B. wilsonae Hemsl.; Mahonia, in particular M. repens and M. aquifolia; Laurelia, in particular L. sempervirens; Cocculus, in particular C. laurifolius; and Xanthorhiza, in particular simplicissima, the parts of the plant used for the preparation of said extract being the leaves, the stems, the roots and the berries and preferably the cortex of the stems or roots.

**19.** The use according to one of claims 14 to 18, characterized in that the above-mentioned extract is an extract of Berberis with the exclusion of Berberis thunbergii, preferably selected from B. vulgaris, B. cretica, B. pseudambalata, B. stolonifera and B. laurina, in particular an extract of the root cortex of this plant.

**20.** The use according to any one of claims 14 to 19, characterized in that the concentration by weight of oxyacanthine or one of its above-mentioned acid addition salts is between 0.001% and 5%, preferably between 0.01% and 2%, based on the total weight of the composition.

**21.** The use according to any one of claims 14 to 20, characterized in that the above-mentioned saponin is selected from the group comprising the steroid saponins, especially the saponins containing sapogenin of the "diosgenin" type and those containing sapogenin of the "sarsapogenin" type, and the triterpene saponins, especially the saponins containing sapogenin of the "oleanane" structure, such as oleanolic acid, medicagenic acid, sapogenol or hederagenin, and those containing sapogenin of the "ursane" structure, such as ursolic acid or asiatic acid.

**22.** The use according to any one of claims 14 to 21, characterized in that the above-mentioned saponin is extracted from one of the following plants: Tribulus terrestris, Dioscorea, Smilax excelsa, Paris polyphylla, Cornus florida, Yucca, Smilax aristolochiaefolia, Asparagus officinalis, Hedera helix, Medicago - in particular Medicago sativa or lucerne - Centella asiatica and Trigonella fenugraecum.

**23.** The use according to claim 22, characterized in that it relates to a mixture of saponins extracted from one of the above-mentioned plants, in particular from Medicago sativa.

**24.** The use according to one of claims 14 to 23, characterized in that the relative proportions of oxyacanthine, one of its acid addition salts or a plant extract in which it is present, and of a saponin or a mixture of saponins, as mentioned above, are between 10/1 and 1/10 by weight, this ratio preferably is 1/1 by weight.

**25.** The use according to one of claims 14 to 24, characterized in that the above-mentioned composition also comprises an effective concentration of at least one other active substance selected from quinine or its derivatives, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate,

minoxidil, azelaic acid and its derivatives, or a 4-methyl-4-azasteroid, in particular 17-β-N,N-diethylcarbamoyl-4-methyl-4-aza-5-α-androstan-3-one, or else an extract of Serenoa repens.

**26.** The use according to one of claims 14 to 25, characterized in that the above-mentioned composition comprises the association of oxyacanthine, one of its derivatives or salts or an extract in which it is present, such as an extract of B. vulgaris, with minoxidil; the oxyacanthine, its derivatives or salts or the extracts in which it is present are preferably incorporated into hydrated lipidic lamellar phases or into liposomes, the whole being mixed with an excipient compatible with these active products, or else the association is prepared from the separately packaged products, either in the form of a mixture prepared immediately before use, or by substantially simultaneous or successive applications of these products to the areas to be treated.

**27.** A method of cosmetic treatment intended for promoting hair growth, retarding hair loss or combating pruritus, especially pruritus of the scalp, characterized in that it comprises applying, to the area to be treated, an amount, effective for achieving said desired effect, of oxyacanthine, one of its derivatives or salts, of formula (I) given above, one of its acid addition salts or a plant extract in which it is present, with the exclusion of an extract of the plant Berberis thunbergii and of the plant Mahonia.

**28.** A method of cosmetic treatment intended for promoting hair growth, retarding hair loss or combating pruritus, especially pruritus of the scalp, characterized in that it comprises applying, to the area to be treated, an amount, effective for achieving said desired effect, of oxyacanthine, one of its derivatives or salts, of formula (I) given above, one of its acid addition salts or a plant extract in which it is present, in association with at least one saponin.

**29.** A method of cosmetic treatment intended for promoting hair growth, retarding hair loss or combating pruritus, especially pruritus of the scalp, characterized in that it comprises applying, to the area to be treated, an amount, effective for achieving said desired effect, of an active substance consisting of oxyacanthine, one of its derivatives or salts, of formula (I) given above, one of its acid addition salts or a plant extract in which it is present, in the presence of hydrated lipidic lamellar phases or of liposomes containing said active substance.

**30.** The method according to one of claims 27 to 29, characterized in that the above-mentioned oxyacanthine, one of its derivatives, one of their acid addition salts or the plant extract in which it is present is associated with at least one saponin.

**31.** The method according to one of claims 27 to 30, characterized in that the above-mentioned oxyacanthine, one of its derivatives, one of their acid addition salts or the plant extract in which it is present is incorporated into hydrated lipidic lamellar phases or into liposomes.

**32.** The method according to any one of claims 27 to 31, characterized in that a composition containing from 0.001% to 5% by weight of oxyacanthine, one of its derivatives or salts, preferably of formula (I) given above, or a plant extract in which it is present, based on the total weight of the composition, is applied.

**33.** The method according to any one of claims 27 to 31, characterized in that oxyacanthine, one of its derivatives or salts or a plant extract in which it is present, such as an extract of B. vulgaris, is associated with minoxidil, the oxyacanthine, its derivatives or salts or the extracts in which it is present preferably is incorporated into hydrated lipidic lamellar phases or into liposomes, the whole being mixed with an excipient compatible with these active products, or else the association is produced from the separately packaged products, either in the form of a mixture prepared immediately before use, or by substantially simultaneous or successive applications of these products to the areas to be treated.

**Patentansprüche**

**1.** Kosmetische Zusammensetzung zur Stimulation des Haarwuchses, zur Verlangsamung von Haarausfall oder zur Bekämpfung von Pruritus, insbesondere Pruritus der Kopfhaut, dadurch gekennzeichnet, daß sie, als aktiven Bestandteil, umfaßt: eine kosmetisch wirksame Menge an Oxyakanthin, oder eines seiner Derivate oder Salze, vorzugsweise der folgenden Formel (I):

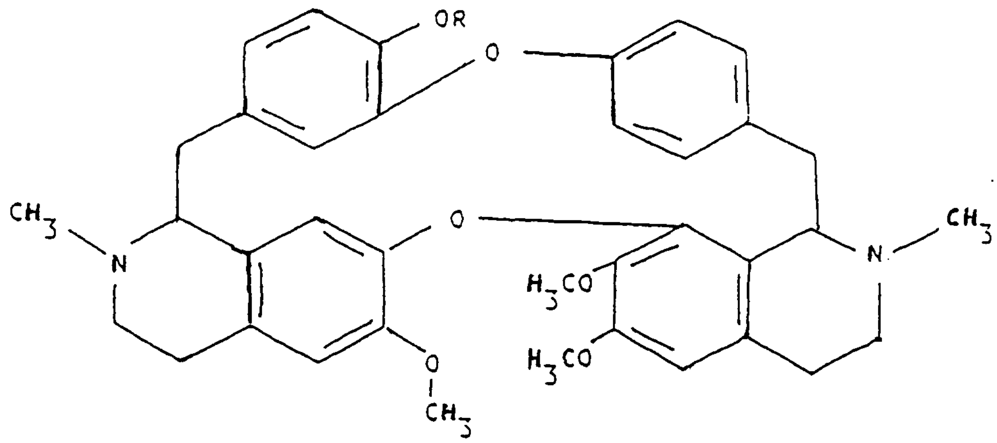

(I),

worin R Wasserstoff (Oxyakanthin), eine $C_1$-$C_{12}$-Kohlenwasserstoffkette oder einen $C_2$-$C_{12}$-Acylrest bedeutet, wobei die Kohlenwasserstoffkette und der Acylrest gesättigt oder ungesättigt, linear, verzweigt oder teilweise cyclisch, insbesondere aromatisch sein können; R insbesondere einen Methyl-, Hexyl-, Decyl-, Acetly-, Propionyl-, Butanoyl-, Pentanoyl-, Hexanoyl-, Octanoyl-, bevorzugter Methyl-, Acetyl- oder Propionylrest darstellt; eines seiner insbesondere kosmetisch annehmbaren Säureadditionssalze; oder eines Pflanzen-Extrakts, der Oxyakanthin oder eines seiner Derivate oder Salze enthält, mit Ausnahme eines Extrakts der Pflanze Berberis thunbergii, und der Pflanze Mahonia.

2. Kosmetische oder pharmazeutische, insbesondere dermatologische Zusammensetzung zur Stimulation des Haarwuchses, zur Verlangsamung von Haarausfall oder zur Bekämpfung von Pruritus, insbesondere Pruritus der Kopfhaut, dadurch gekennzeichnet, daß sie, als aktiven Bestandteil, umfaßt: eine kosmetisch oder pharmazeutisch wirksame Menge einer Vereinigung, die aus Oxyakanthin, einem seiner Derivate oder Salze, vorzugsweise der Formel (I), oder einem seiner insbesondere kosmetisch oder pharmazeutisch annehmbaren Säureadditionssalze, oder einem diese enthaltenden Pflanzen-Extrakt und zumindest einem Saponin besteht.

3. Kosmetische oder pharmazeutische, insbesondere dermatologische Zusammensetzung zur Stimulation des Haarwuchses, zur Verlangsamung von Haarausfall oder zur Bekämpfung von Pruritus, insbesondere Pruritus der Kopfhaut, dadurch gekennzeichnet, daß sie, als aktiven Bestandteil, umfaßt: eine kosmetisch oder pharmazeutisch wirksame Menge einer aktiven Substanz, die aus Oxyakanthin, einem seiner Derivate oder Salze, vorzugsweise der Formel (I), oder einem seiner insbesondere kosmetisch oder pharmazeutisch annehmbaren Säureadditionssalze, oder einem diese enthaltenden Pflanzen-Extrakt besteht, in Anwesenheit von hydratisierten lamellaren Lipidphasen oder Liposomen, welche die aktive Substanz gegebenenfalls einschließen.

4. Zusammensetzung nach einem der Ansprüche 1 bis 3, dadurch gekennzeichnet, daß sie Oxyakanthinsulfat oder eine Mischung von Sulfaten tertiärer Basen, die Oxyakanthinsulfat enthalten, umfaßt, wobei diese Mischung allgemein aus einem Pflanzen-Extrakt hergestellt wird.

5. Zusammensetzung nach einem der Ansprüche 1 bis 4, dadurch gekennzeichnet, daß der Pflanzen-Extrakt ein Extrakt einer Pflanze ist, die aus den folgenden Arten ausgewählt ist: Berberis, insbesondere B. amurensis Rupr., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib, B. laurina, B. nummularia, B. oblonga, B. orthobotrys, B. paucidentata Rusby, B. polyantha Hemsl., B. poiretii Schneid., B. pseudambalata, B. sargentiana Schneid., B. silva-taroucana Schneid., B. soulieana Schneid., B. stolonifera, B. vernae Schneid., B. vulgaris oder Berberitze, B. wilsonae Hemsl.; Mahonia, insbesondere M. repens, M. aquifolia; Laurelia, insbesondere L. sempervirens; Cocculus, insbesondere C. laurifolius; Xanthorhiza, insbesondere X. simplicissima, wobei die zur Herstellung des Extrakts verwendete Teile der Pflanze die Blätter, die Stiele, die Wurzeln, die Beeren und vorzugsweise die Rinde der Stiele oder der Wurzeln sind.

6. Zusammensetzung nach einem der Ansprüche 1 bis 4, dadurch gekennzeichnet, daß der Extrakt ein Extrakt von Berberis ist, mit Ausnahme von Berberis thunbergii, vorzugsweise ausgewählt aus B. vulgaris, B. cretica, B. pseudambalata, B. stolonifera, B. laurina, insbesondere ein Extrakt der Wurzelrinde dieser Pflanze.

7. Zusammensetzung nach einem der Ansprüche 1 bis 6, dadurch gekennzeichnet, daß die Masse-Konzentration von Oxyakanthin, eines seiner Derivate oder eines seiner Säureadditionssalze, bezogen auf die Gesamtmasse der Zusammensetzung, zwischen 0,001 % und 5 %, vorzugsweise zwischen 0,01 % und 2 % liegt.

8. Zusammensetzung nach einem der Ansprüche 2 und 4 bis 7, dadurch gekennzeichnet, daß das Saponin ausgewählt ist aus der Gruppe von Steroid-Saponinen, insbesondere den Saponinen von Sapogenin vom Typ "Diosgenin" und jenen von Sapogenin vom Typ "Sarsapogenin", und von Triterpen-Saponinen, insbesondere den Saponinen von Sapogenin mit "Oleanan"-Struktur, wie Oleanolsäure, Medicagensäure, Sapogenol oder Hederagenin, und jenen von Sapogenin mit "Ursan"-Struktur, wie Ursolsäure oder Asiatinsäure.

9. Zusammensetzung nach einem der Ansprüche 2 und 4 bis 8, dadurch gekennzeichnet, daß das Saponin aus einer der folgenden Pflanzen extrahiert wird: Tribulus terrestris, Dioscorea, Smilax excelsa, Paris polyphylla, Cornus florida, Yucca, Smilax aristolochiaefolia, Asparagus officinalis, Hedera helix, Medicago, insbesondere Medicago sativa oder Luzerne, Centella asiatica, Trigonella fenugraecum.

10. Zusammensetzung nach Anspruch 9, dadurch gekennzeichnet, daß sie eine Mischung von Saponinen enthält, die aus einer der Pflanzen, insbesondere Medicago sativa, extrahiert werden.

11. Zusammensetzung nach einem der Ansprüche 2 und 4 bis 10, dadurch gekennzeichnet, daß die relativen Mengen an Oxyakanthin oder eines seiner Säureadditionssalze oder eines diese enthaltenden Pflanzen-Extrakts und eines Saponins oder einer Mischung von Saponinen zwischen 10/1 und 1/10, bezogen auf die Masse, liegen, wobei dieses Verhältnis vorzugsweise 1/1, bezogen auf die Masse, beträgt.

12. Zusammensetzung nach einem der Ansprüche 1 bis 11, dadurch gekennzeichnet, daß sie außerdem umfaßt: zumindest eine andere aktive Substanz, in einer wirksamen Konzentration, ausgewählt aus Chinin oder seinen Derivaten, Rubefazientien, wie Methylnicotinat, einem Papillenfibroblasten-Kulturüberstand, Keratinhydrolysaten, Spurenelementen, wie Zink, Seien, Kupfer, 5-$\alpha$-Reduktase-Inhibitoren, wie Progesteron, Cyproteronacetat, Minoxidil, Azelainsäure und ihren Derivaten, einem 4-Methyl-4-azasteroid, insbesondere 17-$\beta$-N,N-Diethylcarbamoyl-4-methyl-4-aza-5-$\alpha$-androstan-3-on, oder auch einen Extrakt von Serenoa repens.

13. Zusammensetzung nach einem der Ansprüche 1 bis 12, dadurch gekennzeichnet, daß sie umfaßt: eine Vereinigung von Oxyakanthin, eines seiner Derivate oder Salze, oder eines diese enthaltenden Extrakts, wie eines Extrakts von B. vulgaris, mit Minoxidil, wobei vorzugsweise das Oxyakanthin, seine Derivate oder Salze, oder die diese enthaltenden Extrakte in hydratisierten lamellaren Lipidphasen oder Liposomen eingeschlossen sind, das Ganze in einem mit diesen aktiven Produkten kompatiblen Exzipienten gemischt wird, oder auch die Vereinigung aus getrennt konditionierten Produkten hergestellt wird, entweder in Form einer unmittelbar bei der Verwendung zubereiteten Mischung, oder durch im wesentlichen gleichzeitiges oder aufeinanderfolgendes Aufbringen dieser Produkte auf die zu behandelnden Zonen.

14. Verwendung, als aktiven Bestandteil, von Oxyakanthin, eines seiner Derivate oder Salze, vorzugsweise der Formel (I), eines seiner kosmetisch oder pharmazeutisch annehmbaren Säureadditionssalze, oder eines diese enthaltenden Pflanzen-Extrakts, mit Ausnahme eines Extrakts der Pflanze Berberis thunbergii, und der Pflanze Mahonia, bei der Herstellung einer pharmazeutischen, insbesondere dermatologischen Zusammensetzung zur Stimulation des Haarwuchses, zur Verlangsamung von Haarausfall oder zur Bekämpfung von Pruritus, insbesondere Pruritus der Kopfhaut; oder als kosmetisches Mittel zur Stimulation des Haarwuchses, zur Verlangsamung von Haarausfall oder zur Bekämpfung von Pruritus, insbesondere Pruritus der Kopfhaut.

15. Verwendung, als aktiven Bestandteil, von Oxyakanthin, eines seiner Derivate oder Salze, vorzugsweise der Formel (I), eines seiner kosmetisch oder pharmazeutisch annehmbaren Säureadditionssalze, oder eines diese enthaltenden Pflanzen-Extrakts, in Vereinigung mit zumindest einem Saponin, bei der Herstellung einer pharmazeutischen, insbesondere dermatologischen Zusammensetzung zur Stimulation des Haarwuchses, zur Verlangsamung von Haarausfall oder zur Bekämpfung von Pruritus, insbesondere Pruritus der Kopfhaut; oder als kosmetisches Mittel zur Stimulation des Haarwuchses, zur Verlangsamung von Haarausfall oder zur Bekämpfung von Pruritus, insbesondere Pruritus der Kopfhaut.

16. Verwendung einer aktiven Substanz, die aus Oxyakanthin, einem seiner Derivate oder Salze, vorzugsweise der Formel (I), einem seiner kosmetisch oder pharmazeutisch annehmbaren Säureadditionssalze, oder einem diese enthaltenden Pflanzen-Extrakt besteht, in Anwesenheit hydratisierter lamellarer Lipidphasen oder Liposomen, welche die aktive Substanz gegebenenfalls einschließen, bei der Herstellung einer pharmazeutischen, insbesondere

dermatologischen Zusammensetzung zur Stimulation des Haarwuchses, zur Verlangsamung von Haarausfall oder zur Bekämpfung von Pruritus, insbesondere Pruritus der Kopfhaut; oder als kosmetisches Mittel zur Stimulation des Haarwuchses, zur Verlangsamung von Haarausfall oder zur Bekämpfung von Pruritus, insbesondere Pruritus der Kopfhaut.

**17.** Verwendung nach einem der Ansprüche 14 bis 16, dadurch gekennzeichnet, daß der aktive Bestandteil Oxyakanthinsulfat oder eine Mischung von Sulfaten tertiärer Basen, die Oxyakanthinsulfat enthalten, ist, wobei diese Mischung allgemein aus einem Pflanzen-Extrakt hergestellt wird.

**18.** Verwendung nach einem der Ansprüche 14 bis 17, dadurch gekennzeichnet, daß der Pflanzen-Extrakt ein Extrakt einer Pflanze ist, die aus den folgenden Arten ausgewählt ist: Berberis, insbesondere B. amurensis Rupr., B. boliviana Lechl., B. bumeliaefolia Schneid., B. buxifolia, B. chingii Cheng., B. coriaria, B. cretica, B. dielsiana Fedde., B. integerrima, B. julianea Schneid., B. koreana Palib, B. laurina, B. nummularia, B. oblonga, B. orthobotrys, B. paucidentata Rusby, B. polyantha Hemsl., B. poiretii Schneid., B. pseudambalata, B. sargentiana Schneid., B. silvataroucana Schneid., B. soulieana Schneid., B. stolonifera, B. vernae Schneid., B. vulgaris oder Berberitze, B. wilsonae Hemsl.; Mahonia, insbesondere M. repens, M. aquifolia; Laurelia, insbesondere L. sempervirens; Cocculus, insbesondere C. laurifolius; Xanthorhiza, insbesondere X. simplicissima, wobei die zur Herstellung des Extrakts verwendete Teile der Pflanze die Blätter, die Stiele, die Wurzeln, die Beeren und vorzugsweise die Rinde der Stiele oder Wurzeln sind.

**19.** Verwendung nach einem der Ansprüche 14 bis 18, dadurch gekennzeichnet, daß der Extrakt ein Extrakt von Berberis ist, mit Ausnahme von Berberis thunbergii, vorzugsweise ausgewählt aus B. vulgaris, B. cretica, B. pseudambalata, B. stolonifera, B. laurina, insbesondere ein Extrakt der Wurzelrinde dieser Pflanze.

**20.** Verwendung nach einem der Ansprüche 14 bis 19, dadurch gekennzeichnet, daß die Masse-Konzentration von Oxyakanthin oder einem seiner Säureadditionssalze, bezogen auf die Gesamtmasse der Zusammensetzung, zwischen 0,001 % und 5 %, vorzugsweise zwischen 0,01 % und 2 % liegt.

**21.** Verwendung nach einem der Ansprüche 14 bis 20, dadurch gekennzeichnet, daß das Saponin ausgewählt ist aus der Gruppe von steroiden Saponinen, insbesondere den Saponinen von Sapogenin vom Typ "Diosgenin" und jenen von Sapogenin vom Typ "Sarsapogenin", und den Triterpen-Saponinen, insbesondere den Saponinen von Sapogenin mit "Oleanan"-Struktur, wie Oleanolsäure, Medicagensäure, Sapogenol oder Hederagenin, und jenen von Sapogenin mit "Ursan"-Struktur, wie Ursolsäure oder Asiatinsäure.

**22.** Verwendung nach einem der Ansprüche 14 bis 21, dadurch gekennzeichnet, daß das Saponin aus einer der folgenden Pflanzen extrahiert wird: Tribulus terrestris, Dioscorea, Smilax excelsa, Paris polyphylla, Cornus florida, Yucca, Smilax aristolochiaefolia, Asparagus officinalis, Hedera helix, Medicago, insbesondere Medicago sativa oder Luzerne, Centella asiatica, Trigonella fenugraecum.

**23.** Verwendung nach Anspruch 22, dadurch gekennzeichnet, daß sie eine Mischung von Saponinen betrifft, die aus einer der Pflanzen, insbesondere Medicago sativa, extrahiert werden.

**24.** Verwendung nach einem der Ansprüche 14 bis 23, dadurch gekennzeichnet, daß die relativen Mengen an Oxyakanthin oder eines seiner Säureadditionssalze oder eines diese enthaltenden Pflanzen-Extrakts und eines Saponins oder einer Mischung von Saponinen zwischen 10/1 und 1/10, bezogen auf die Masse, liegen, wobei dieses Verhältnis vorzugsweise 1/1, bezogen auf die Masse, beträgt.

**25.** Verwendung nach einem der Ansprüche 14 bis 24, dadurch gekennzeichnet, daß die Zusammensetzung außerdem umfaßt: zumindest eine andere aktive Substanz, in einer wirksamen Konzentration, ausgewählt aus Chinin oder seinen Derivaten, Rubefazientien, wie Methylnicotinat, einem Papillenfibroblasten-Kulturüberstand, Keratinhydrolysaten, Spurenelementen, wie Zink, Seien, Kupfer, 5-$\alpha$-Reduktase-Inhibitoren, wie Progesteron, Cyproteronacetat, Minoxidil, Azelainsäure und ihren Derivaten, einem 4-Methyl-4-azasteroid, insbesondere 17-$\beta$-N,N-Diethylcarbamoyl-4-methyl-4-aza-5-$\alpha$-androstan-3-on, oder auch einen Extrakt von Serenoa repens.

**26.** Verwendung nach einem der Ansprüche 14 bis 25, dadurch gekennzeichnet, daß die Zusammensetzung umfaßt: eine Vereinigung von Oxyakanthin, eines seiner Derivate oder Salze, oder eines diese enthaltenden Extrakts, wie eines Extrakts von B. vulgaris, mit Minoxidil, wobei vorzugsweise das Oxyakanthin, seine Derivate oder Salze, oder die diese enthaltenden Extrakte in hydratisierten lamellaren Lipidphasen oder Liposomen eingeschlossen sind, das Ganze in einem mit diesen aktiven Produkten kompatiblen Exzipienten gemischt wird, oder auch die Vereinigung aus getrennt konditionierten Produkten hergestellt wird, entweder in Form einer unmittelbar bei der Verwendung zubereiteten Mischung, oder durch im wesentlichen gleichzeitiges oder aufeinanderfolgendes Aufbringen dieser Produkte auf die zu behandelnden Zonen.

**27.** Verfahren zur kosmetischen Behandlung zur Förderung des Haarwuchses, zur Verlangsamung von Haarausfall oder zur Bekämpfung von Pruritus, insbesondere Pruritus der Kopfhaut, dadurch gekennzeichnet, daß es das Aufbringen einer zum Erzielen des gewünschten Effekts wirksamen Menge an Oxyakanthin, eines seiner Derivate oder Salze, der Formel (I), eines seiner Säureadditionssalze oder eines diese enthaltenen Pflanzenextrakts, mit Ausnahme eines Extrakts von Berberis thunbergii, und Mahonia, auf die zu behandelnde Zone umfaßt.

**28.** Verfahren zur kosmetischen Behandlung zur Förderung des Haarwuchses, zur Verlangsamung von Haarausfall oder zur Bekämpfung von Pruritus, insbesondere Pruritus der Kopfhaut, dadurch gekennzeichnet, daß es das Aufbringen einer zum Erzielen des gewünschten Effekts wirksamen Menge an Oxyakanthin, eines seiner Derivate oder Salze, der Formel (I), eines seiner Säureadditionssalze oder eines diese enthaltenen Pflanzenextrakts in Vereinigung mit zumindest einem Saponin auf die zu behandelnde Zone umfaßt.

**29.** Verfahren zur kosmetischen Behandlung zur Förderung des Haarwuchses, zur Verlangsamung von Haarausfall oder zur Bekämpfung von Pruritus, insbesondere Pruritus der Kopfhaut, dadurch gekennzeichnet, daß es das Aufbringen, auf die zu behandelnde Zone, einer zum Erzielen des gewünschten Effekts wirksamen Menge einer aktiven Substanz umfaßt, die aus Oxyakanthin, einem seiner Derivate oder Salze, der Formel (I), einem seiner Säureadditionssalze oder einem diese enthaltenden Pflanzen-Extrakt besteht, in Anwesenheit von hydratisierten lamellaren Lipidphasen oder Liposomen, welche die aktive Substanz enthalten.

**30.** Verfahren nach einem der Ansprüche 27 bis 29, dadurch gekennzeichnet, daß Oxyakanthin, eines seiner Derivate, eines seiner Säureadditionssalze oder ein diese enthaltender Pflanzen-Extrakt mit zumindest einem Saponin vereinigt wird.

**31.** Verfahren nach einem der Ansprüche 27 bis 30, dadurch gekennzeichnet, daß Oxyakanthin, eines seiner Derivate, eines seiner Säureadditionssalze oder ein diese enthaltender Pflanzen-Extrakt in die hydratisierten lamellaren Lipidphasen oder Liposomen eingeschlossen wird.

**32.** Verfahren nach einem der Ansprüche 27 bis 31, dadurch gekennzeichnet, daß eine Zusammensetzung, die 0,001 Masse-% bis 5 Masse-% Oxyakanthin, eines seiner Derivate oder Salze, vorzugsweise der Formel (I), oder eines diese enthaltenden Pflanzen-Extrakts umfaßt, bezogen auf die Gesamtmasse der Zusammensetzung, aufgebracht wird.

**33.** Verfahren nach einem der Ansprüche 27 bis 32, dadurch gekennzeichnet, daß Oxyakanthin, eines seiner Derivate oder Salze, oder ein diese enthaltender Pflanzen-Extrakt, wie ein Extrakt von B. vulgaris, mit Minoxidil vereinigt wird, wobei vorzugsweise das Oxyakanthin, seine Derivate oder Salze, oder die diese enthaltenden Extrakte in die hydratisierten lamellaren Lipidphasen oder Liposomen eingeschlossen werden, das Ganze in einem mit diesen aktiven Produkten kompatiblen Exzipienten gemischt wird, oder auch die Vereinigung aus getrennt konditionierten Produkten hergestellt wird, entweder in Form einer unmittelbar bei der Verwendung zubereiteten Mischung oder durch im wesentlichen gleichzeitiges oder aufeinanderfolgendes Aufbringen dieser Produkte auf die zu behandelnden Zonen.

FIG.1

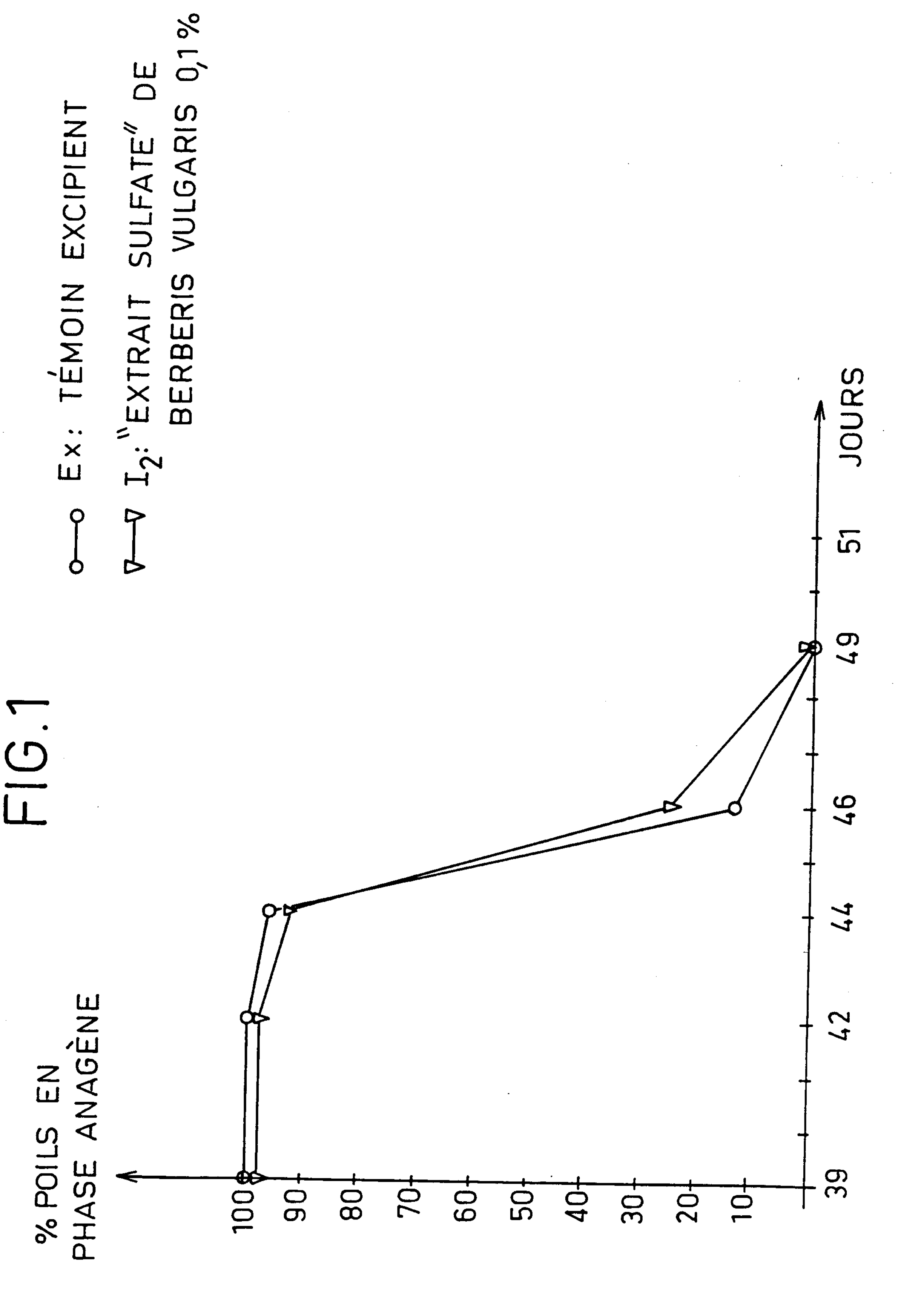

% POILS EN
PHASE ANAGÈNE
100
90
80
70
60
50
40
30
20
10
39
42
44
46
49
51
JOURS
Ex : TÉMOIN EXCIPIENT
$I_2$: "EXTRAIT SULFATE" DE
BERBERIS VULGARIS 0,1 %

FIG. 2

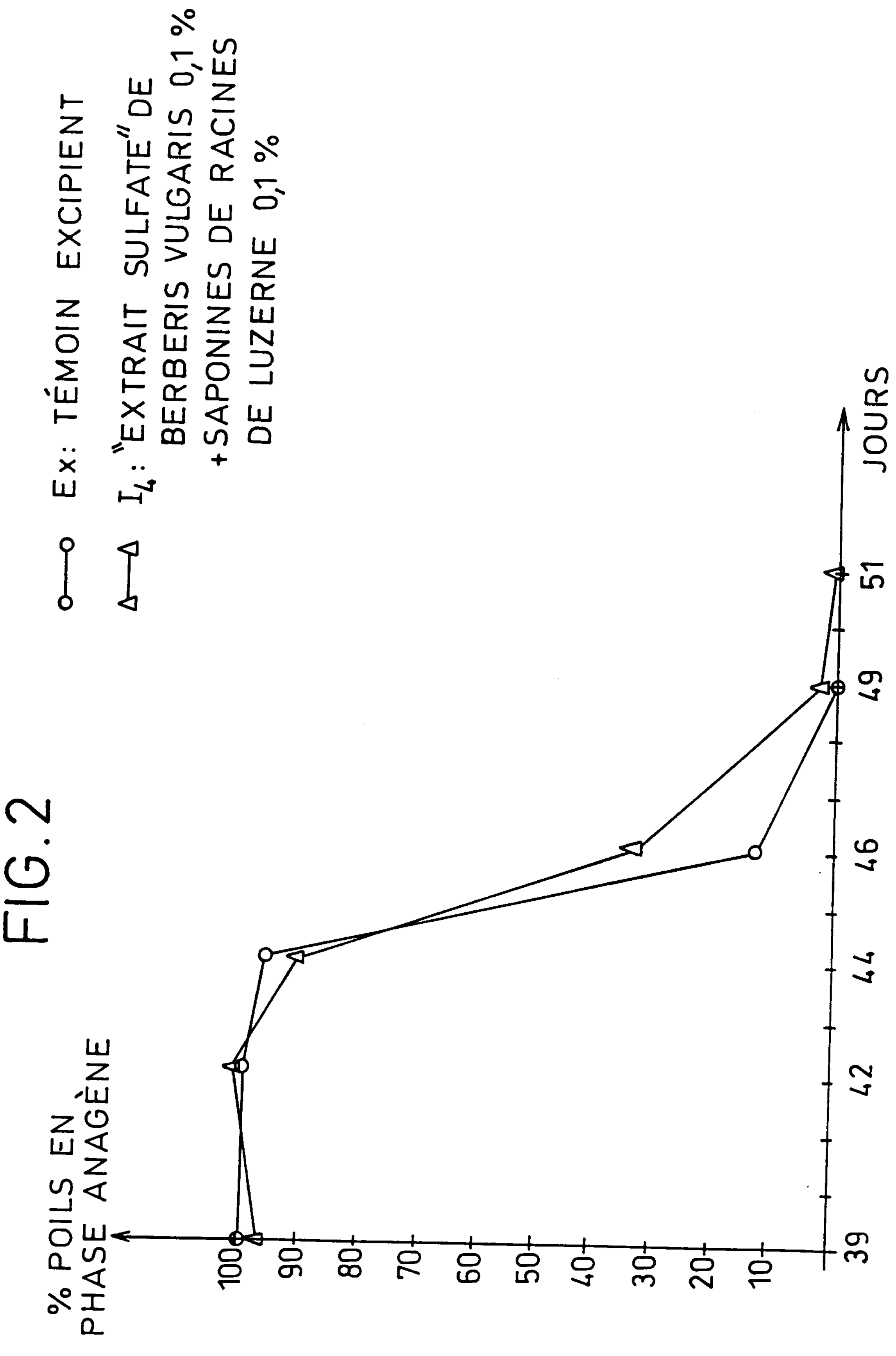
% POILS EN
PHASE ANAGÈNE
100
90
80
70
60
50
40
30
20
10
39
42
44
46
49
51
JOURS
Ex : TÉMOIN EXCIPIENT
I4 : "EXTRAIT SULFATE" DE
BERBERIS VULGARIS 0,1 %
+ SAPONINES DE RACINES
DE LUZERNE 0,1 %

FIG. 3

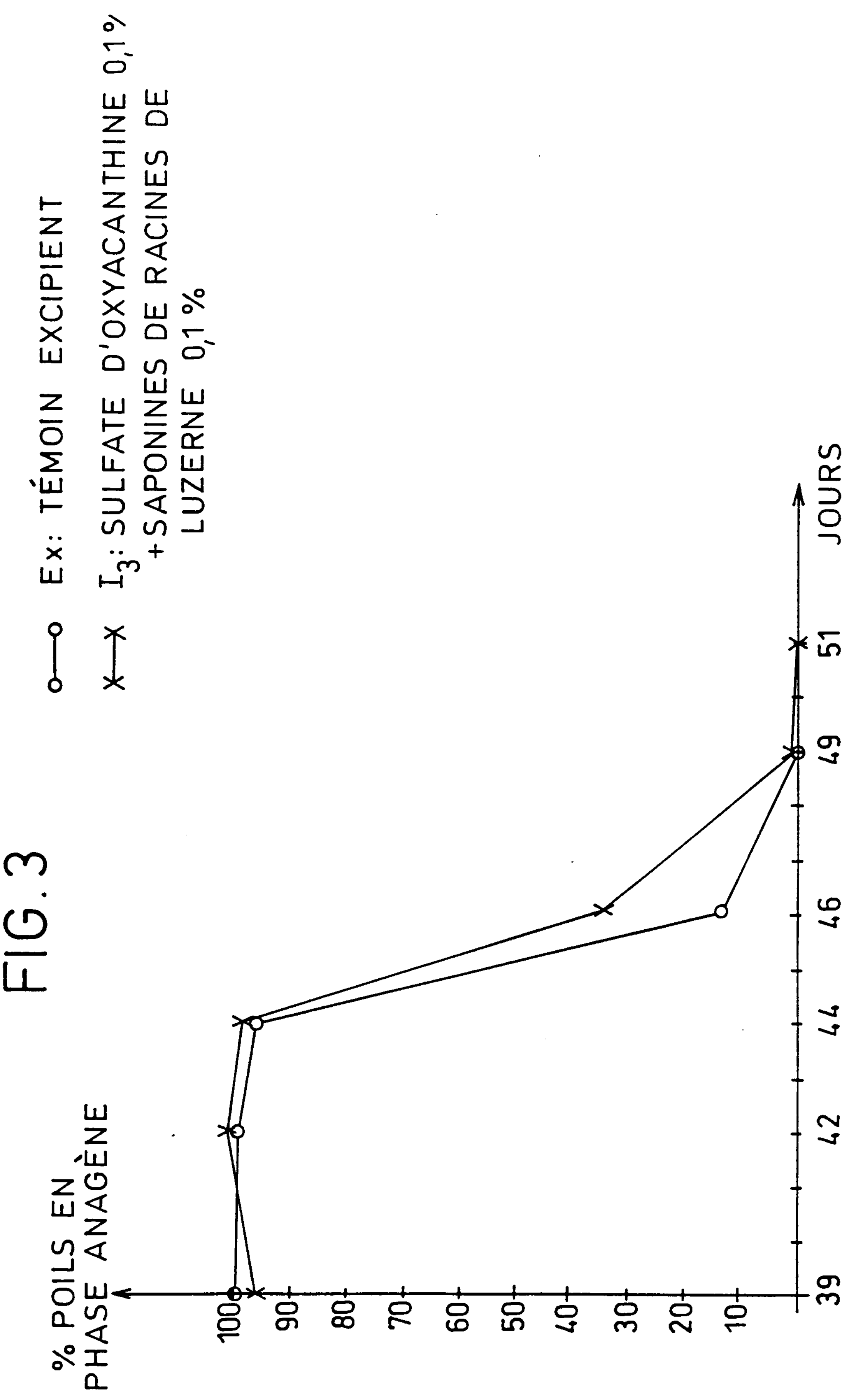
% POILS EN
PHASE ANAGÈNE
100
90
80
70
60
50
40
30
20
10
39
42
44
46
49
51
JOURS
Ex: TÉMOIN EXCIPIENT
$I_3$: SULFATE D'OXYACANTHINE 0,1%
+SAPONINES DE RACINES DE
LUZERNE 0,1%

FIG. 4

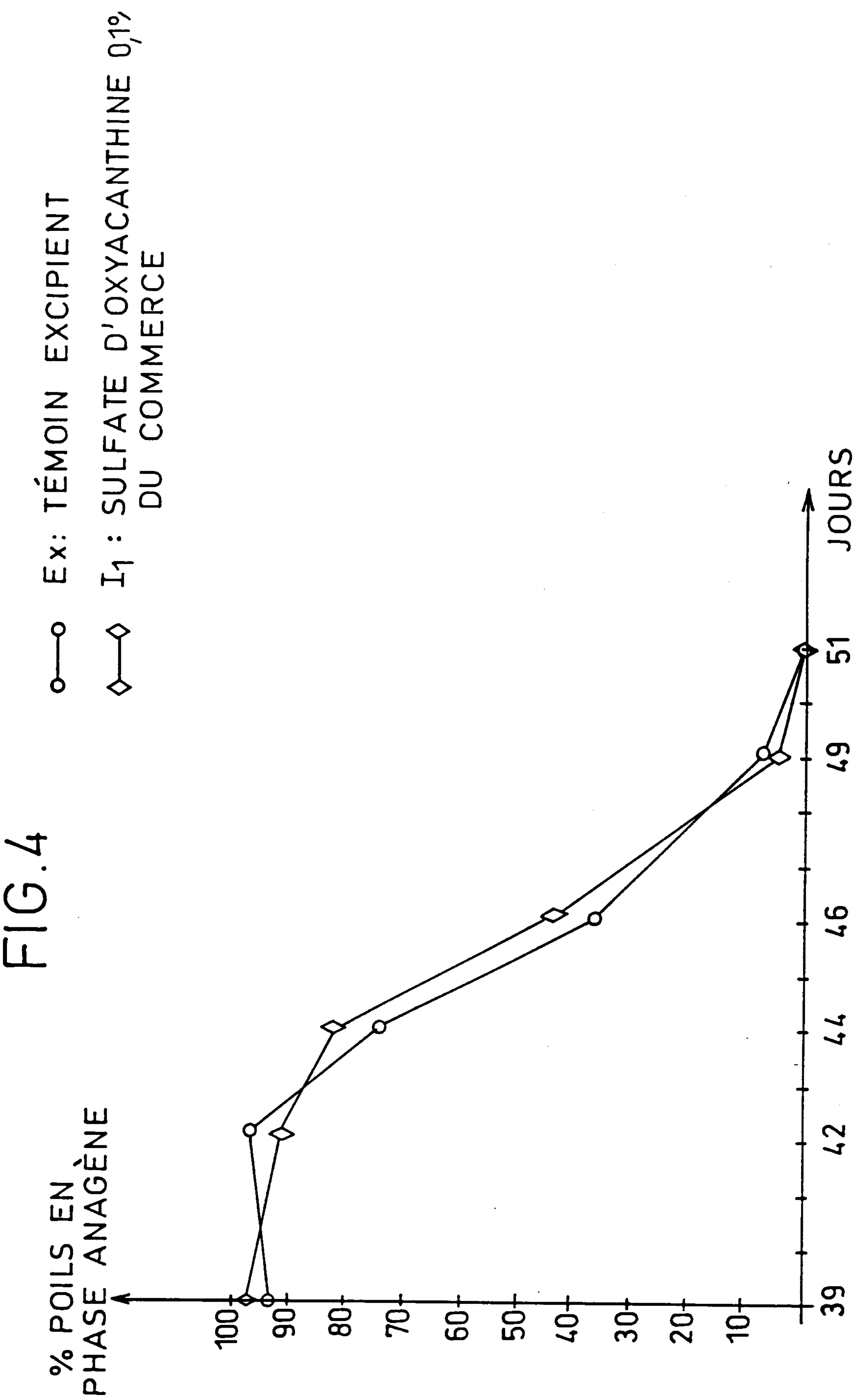
% POILS EN
PHASE ANAGÈNE
100
90
80
70
60
50
40
30
20
10
39
42
44
46
49
51
JOURS
Ex: TÉMOIN EXCIPIENT
I1 : SULFATE D'OXYACANTHINE 0,1%
DU COMMERCE

FIG.5

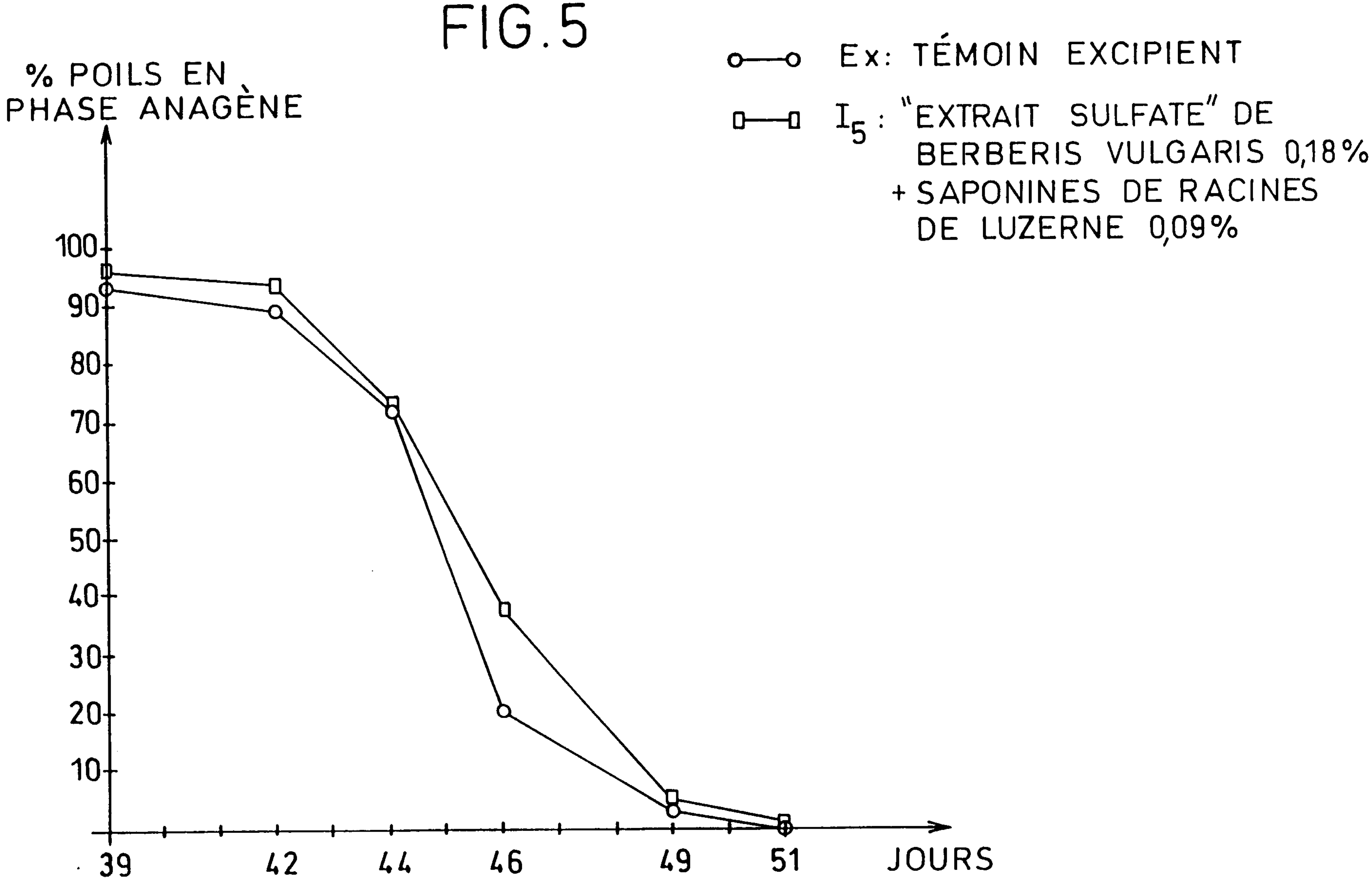
% POILS EN
PHASE ANAGÈNE
100
90
80
70
60
50
40
30
20
10
39
42
44
46
49
51
JOURS
Ex: TÉMOIN EXCIPIENT
I5 : "EXTRAIT SULFATE" DE
BERBERIS VULGARIS 0,18%
+ SAPONINES DE RACINES
DE LUZERNE 0,09%

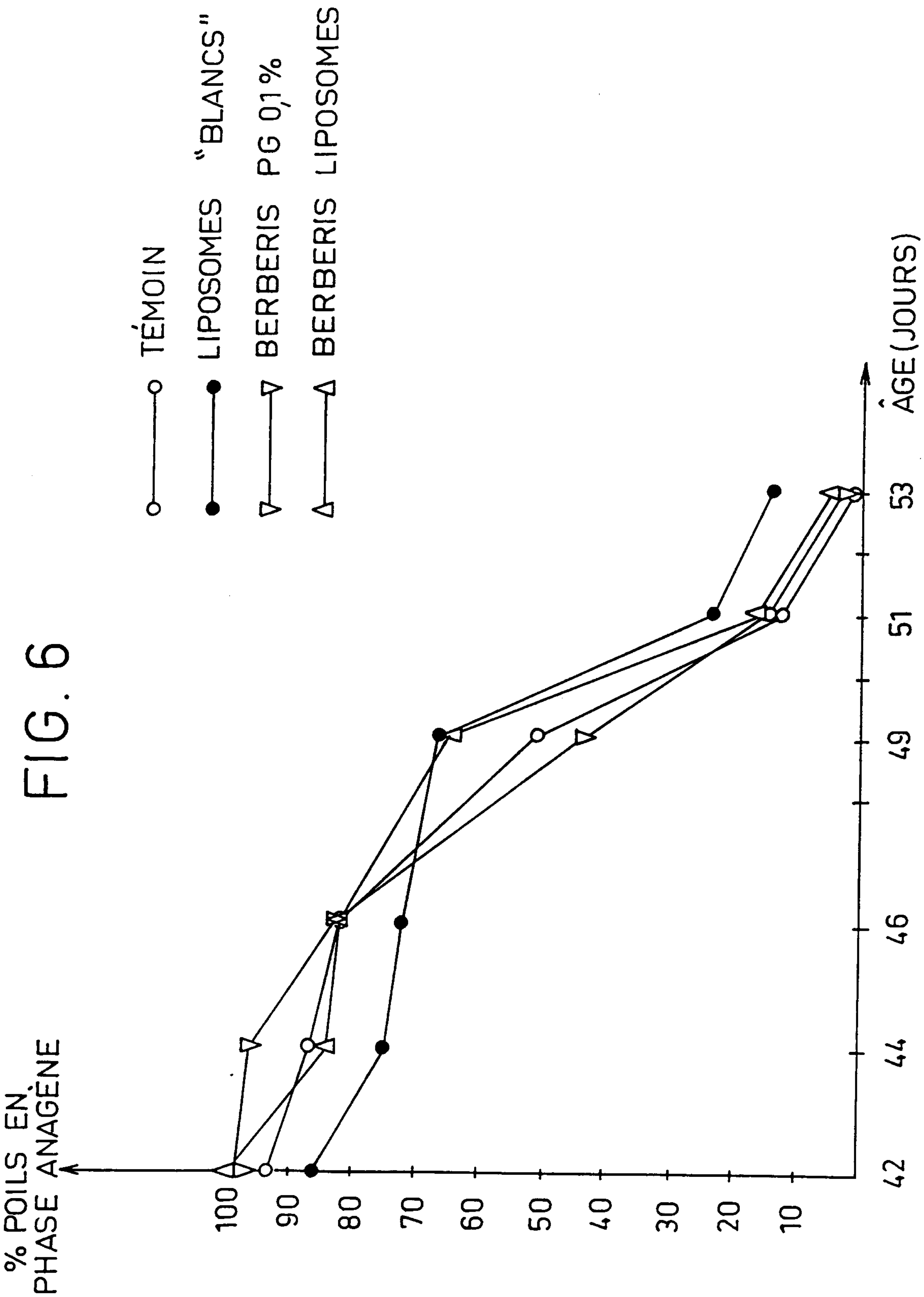

% POILS EN PHASE ANAGÈNE
100
90
80
70
60
50
40
30
20
10
42
44
46
49
51
53
ÂGE (JOURS)
TÉMOIN
LIPOSOMES "BLANCS"
BERBERIS PG 0,1 %
BERBERIS LIPOSOMES


FIG. 6

FIG. 7

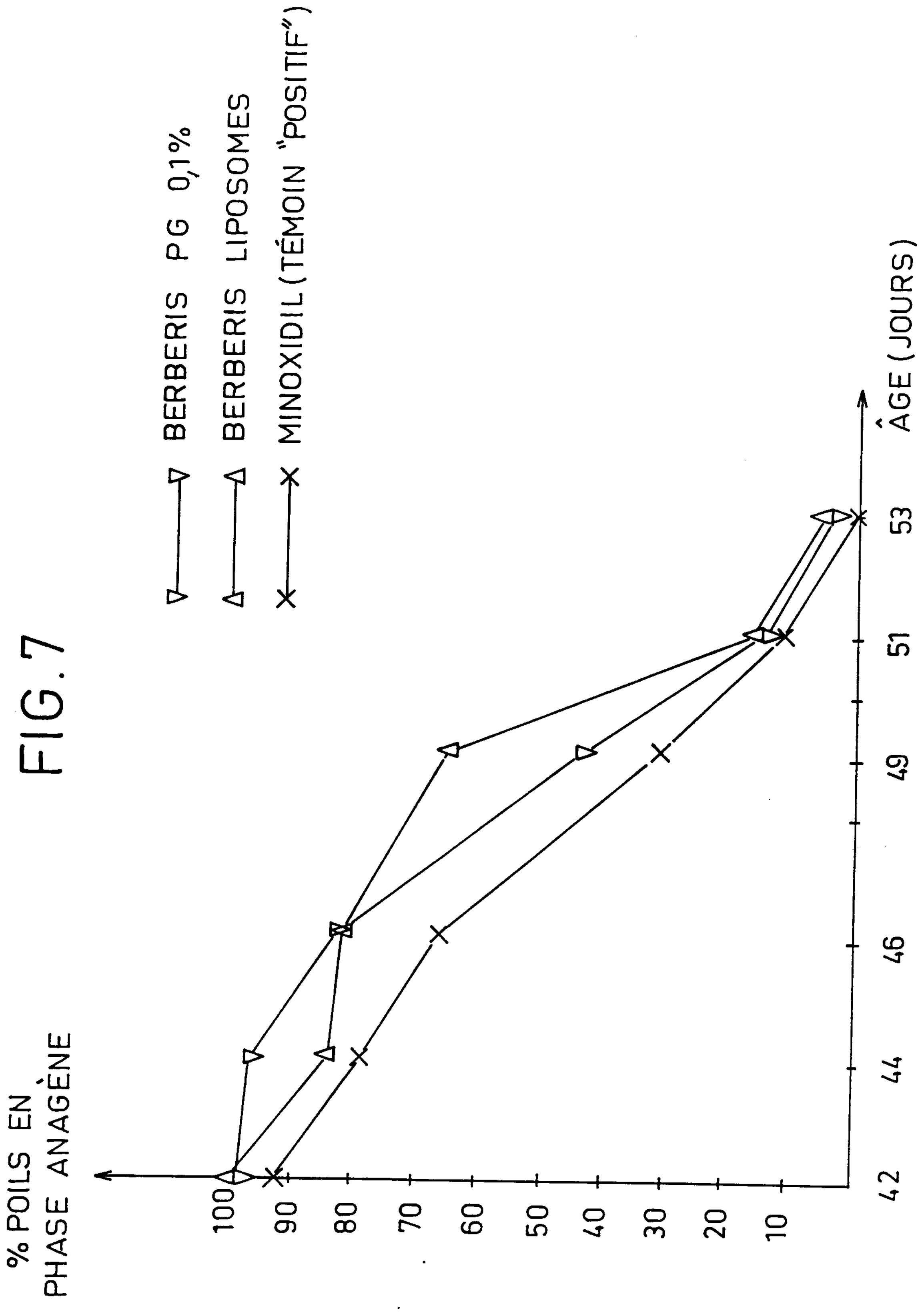
% POILS EN
PHASE ANAGÈNE
100
90
80
70
60
50
40
30
20
10
42
44
46
49
51
53
ÂGE (JOURS)
BERBERIS PG 0,1%
BERBERIS LIPOSOMES
MINOXIDIL (TÉMOIN "POSITIF")